# EUROPÄISCHE PATENTSCHRIFT

(11) **EP 1 293 506 B1**
(45) Veröffentlichungstag und Bekanntmachung des Hinweises auf die Patenterteilung: **28.09.2005**
(21) Anmeldenummer: 02020115.8
(22) Anmeldetag: 07.09.2002
(51) Int. Cl.: C07D 333/20, A61K 31/381, C07D 307/52, A61K 31/341, C07D 407/06, C07D 295/092, C07D 295/08, C07D 209/08, A61P 1/00

(54) **Neue 1-(1-(Hetero)aryl-1-perhydroxyalkylmethyl)-Piperazinverbindungen, Verfahren zu ihrer Herstellung und diese Verbindungen enthaltende Arzneimittel**
New 1-(1-(Hetero)aryl-1-perhydroxyalkylmethyl)-Piperazine derivatives, process for their preparation and pharmaceutical compositions containing the same
Nouveaux derivés de la 1-(1-(Hetero)aryl-1-perhydroxyalkylmethyl)-Piperazine, procédé pour leur obtention et compositions pharmaceutiques les contenant

(30) Priorität: 13.09.2001 DE 10145044
(43) Veröffentlichungstag der Anmeldung: 19.03.2003
(73) Patentinhaber: Solvay Pharmaceuticals GmbH, 30173 Hannover (DE)
(72) Erfinder: Jasserand, Daniel, 30625 Hannover (DE); Preuschoff, Ulf, 31275 Lehrte/Ahlten (DE); Antel, Jochen, 31848 Bad Münder (DE); David, Samuel, 30559 Hannover (DE); Sann, Holger, 30629 Hannover (DE); Brückner, Reinhard, 30559 Hannover (DE); Reiche, Dania, 29352 Adelheidsdorf (DE); Eeckhout, Christian, 29690 Lindwedel (DE)
(74) Vertreter: Bauriegel, Lutz

(56) Entgegenhaltungen:
- EP-A- 0 428 434
- EP-A- 0 474 561
- EP-A- 0 709 375

## Beschreibung

Die vorliegende Erfindung betrifft neue Tachykininrezeptor-antagonistisch wirksame 1-[1-(Hetero)aryl-1-perhydroxyalkylmethyl]-Piperazinverbindungen sowie diese Verbindungen enthaltende Arzneimittel. Ferner betrifft die Erfindung ein Verfahren zur Herstellung der neuen Piperazinverbindungen.

Zu den Tachykininen werden die natürlich vorkommenden Neuropeptide Substanz P, Neurokinin A und Neurokinin B gezählt. Die Tachykinine wirken als Agonisten von in größeren Säugetieren und Menschen vorkommenden Rezeptoren wie dem Neurokinin (= NK)-1-Rezeptor, dem NK-2-Rezeptor und dem NK-3-Rezeptor. Künstlich hergestellte Tachykininrezeptor-antagonistisch wirksame Verbindungen werden üblicherweise nach ihrem relativen Vermögen klassifiziert, an einen oder mehrere der drei vorgenannten Rezeptor-Subtypen zu binden. Die Tachykinine spielen im physiologischen Geschehen z. B. eine wichtige Rolle bei der Schmerzübertragung, der Emese, bei neurogenen Entzündungen, Harnblasenentzündungen, entzündlichen Gelenkserkrankungen oder bei asthmatischen Beschwerden.

In der Schrift EP 0 428 434 A2 werden aromatische Aminverbindungen beschrieben, welche antagonistische Eigenschaften gegenüber spezifischen Agonisten der NK-1, NK-2 und/oder NK-3-Rezeptoren gezeigt haben.

Die Schrift EP 0 709 375 A2 offenbart neue substituierte 1,2-Ethandiamin-Derivate, welche die pharmakologische Aktivität der als Neurokinine bekannten endogenen Tachykinin-Neuropeptide antagonisieren, insbesondere an den NK-1 und NK-2-Rezeptoren.

Aus der EP 0 474 561 A1 sind bereits u. a. Piperazinderivate bekannt, welche als Antagonisten des NK-2-Rezeptors wirksam sind.

Aus der WO 96/10568 sind weitere Piperazinderivate bekannt, welche als Antagonisten von Tachykininrezeptoren wirken können.

Der vorliegenden Erfindung lag die Aufgabe zu Grunde, neue Wirkstoffe mit Tachykininrezeptor-antagonistischen Eigenschaften und einem verbesserten Wirkprofil bereitzustellen, welche insbesondere zur Behandlung von peripheren Störungen wie funktionellen und entzündlichen Störungen des gastrointestinalen Traktes geeignet sind.

Es wurde nun überraschend gefunden, daß sich eine Gruppe von neuen 1-[1-(Hetero)aryl-1-perhydroxyalkylmethyl]-Piperazinverbindungen durch Tachykininrezeptor-antagonistische, insbesondere NK-2-Rezeptor-antagonistische, Eigenschaften auszeichnet und eine ausgeprägte, auf den peripheren Bereich gerichtete Wirkkomponente aufweist. Demnach erscheint die erfindungsgemäße Gruppe von Verbindungen besonders geeignet zur Behandlung von peripheren Störungen, bei denen Tachykinine, insbesondere Neurokinin A, als Überträgerstoffe beteiligt sind, beispielsweise zur Behandlung und/oder Prophylaxe von funktionellen und entzündlichen Störungen des gastrointestinalen Traktes. Die Bezeichnung (Hetero)aryl soll im Rahmen der vorliegenden Erfindung so verstanden werden, daß sowohl Aryl-, als auch Heteroarylreste umfaßt sein können.

Gegenstand der Erfindung sind neue 1-[1-(Hetero)aryl-1-perhydroxyalkylmethyl]-Piperazinverbindungen der allgemeinen Formel I, 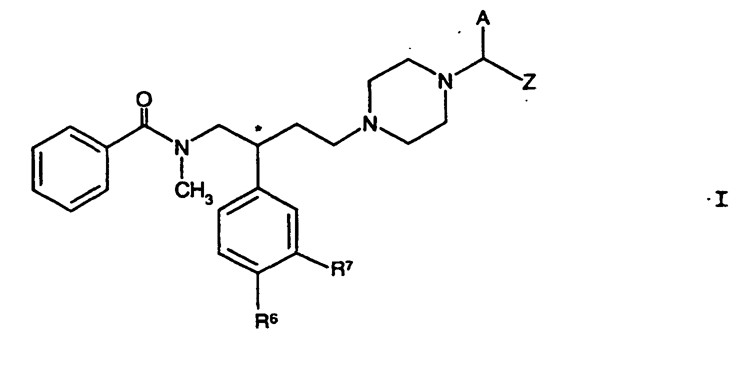 worin
- A: Naphthyl, gegebenenfalls durch Hydroxy substituiertes Phenyl, mono- oder bicyclisches Heteroaryl oder gegebenenfalls durch Phenyl substituiertes C₃₋₆-Alkenyl bedeutet,
- Z: für eine Untergruppe der allgemeinen Formel 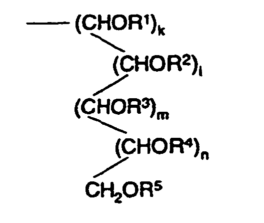 steht, worin
R¹ Wasserstoff oder niederes Alkanoyl bedeutet, oder gemeinsam mit einem anderen Substituenten, ausgewählt aus der Gruppe bestehend aus R², R³, R⁴ und R⁵, einen über Carbonyl, Thiocarbonyl oder über gegebenenfalls durch niederes Alkyl oder C₄₋₅-Alkylen substituiertes Methylen verbrückten 5- oder 6-Ring bilden kann,
R² Wasserstoff oder niederes Alkanoyl bedeutet, oder gemeinsam mit einem anderen Substituenten, ausgewählt aus der Gruppe bestehend aus R¹, R³, R⁴ und R⁵, einen über Carbonyl, Thiocarbonyl oder über gegebenenfalls durch niederes Alkyl oder C₄₋₅-Alkylen substituiertes Methylen verbrückten 5- oder 6-Ring bilden kann,
R³ Wasserstoff oder niederes Alkanoyl bedeutet, oder gemeinsam mit einem anderen Substituenten, ausgewählt aus der Gruppe bestehend aus R¹, R², R⁴ und R⁵, einen über Carbonyl, Thiocarbonyl oder über gegebenenfalls durch niederes Alkyl oder C₄₋₅-Alkylen substituiertes Methylen verbrückten 5- oder 6-Ring bilden kann,
R⁴ Wasserstoff oder niederes Alkanoyl bedeutet, oder gemeinsam mit einem anderen Substituenten, ausgewählt aus der Gruppe bestehend aus R¹, R², R³ und R⁵, einen über Carbonyl, Thiocarbonyl oder über gegebenenfalls durch niederes Alkyl oder C₄₋₅-Alkylen substituiertes Methylen verbrückten 5- oder 6-Ring bilden kann,
R⁵ Wasserstoff oder niederes Alkanoyl bedeutet, oder gemeinsam mit einem anderen Substituenten ausgewählt aus der Gruppe bestehend aus R¹, R², R³ und R⁴, einen über Carbonyl, Thiocarbonyl oder über gegebenenfalls durch niederes Alkyl oder C₄₋₅-Alkylen substituiertes Methylen verbrückten 5- oder 6-Ring bilden kann,
k 0 oder 1 bedeutet,
l 0 oder 1 bedeutet,
m 0 oder 1 bedeutet,
n 0 oder 1 bedeutet,
- R⁶: Halogen oder Wasserstoff bedeutet und
- R⁷: Halogen oder Wasserstoff bedeutet,
sowie physiologisch verträgliche Säureadditionssalze von Verbindungen der Formel I. Weiterhin sind Gegenstand der Erfindung die Verbindungen der Formel I enthaltenden Arzneimittel. Ferner sind Gegenstand der Erfindung ein Verfahren zur Herstellung der Verbindungen der Formel I.

Sofern in den Verbindungen der Formel I oder in anderen im Rahmen der vorliegenden Erfindung beschriebenen Verbindungen Substituenten niederes Alkyl bedeuten oder enthalten, kann dieses jeweils geradkettig oder verzweigt sein und 1 bis 4 Kohlenstoffatome besitzen. Sofern Substituenten in Verbindungen der Formel I für Halogen stehen, kommen Fluor, Chlor oder Brom in Frage. Chlor ist bevorzugt. Sofern Substituenten niederes Alkanoyl enthalten, kann dieses geradkettig oder verzweigt sein und 2 bis 4 Kohlenstoffatome besitzen. Acetyl ist als niederes Alkanoyl bevorzugt.

Die Untergruppe A steht vorzugsweise für monocyclisches Heteroaryl. Als monocyclisches Heteroaryl kommen insbesondere Thiophen, Furan und Pyrrol in Frage. Thiophen und Furan sind bevorzugt. Sofern A für bicyclisches Heteroaryl steht, kommen insbesondere Benzothiophen, Benzofuran und Indol in Frage. Sofern A für gegebenenfalls durch Phenyl substituiertes C₃₋₆-Alkenyl steht, kann die Alkenylkette geradkettig oder verzweigt sein und steht insbesondere für 1-Alkenyl.

Sofern ein von der Untergruppe Z umfaßter Substituent aus der Gruppe bestehend aus R¹, R², R³, R⁴ und R⁵ gemeinsam mit einem anderen aus dieser Gruppe ausgewählten Substituenten für einen über gegebenenfalls durch niederes Alkyl oder C₄₋₅-Alkylen substituiertes Methylen verbrückten 5- oder 6-Ring steht, kommen insbesondere über Methylen, 1,1-Dimethylmethylen, 1,1-*spiro*-Tetramethylen-methylen oder 1,1-*spiro*-Pentamethylen-methylen verbrückte 5- oder 6-Ringe in Frage. Entsprechende über Carbonyl verbrückte 5- oder 6-Ringe sind als cyclische Carbonate aufzufassen. Entsprechende über Thiocarbonyl verbrückte 5- oder 6-Ringe sind als cyclische Thiocarbonate aufzufassen. k steht vorzugsweise für 1. n steht vorzugsweise für 0. Z stellt somit vorzugsweise einen gegebenenfalls substituierten 1,2-Diolrest, einen 1,2,3-Triolrest oder einen 1,2,3,4-Tetrolrest dar. Die die Substituenten R¹, R², R³ und R⁴ tragenden Kohlenstoffatome sind asymmetrisch und können jeweils in zwei verschiedenen Konfigurationen auftreten. Dadurch bedingt kann Z in mehreren stereoisomeren Formen auftreten. Die vorliegende Erfindung umfaßt neben den Verbindungen der Formel I, welche Gemische von stereoisomeren Formen der Untergruppe Z enthalten, auch Verbindungen der Formel I, worin isomerenreine Untergruppen Z enthalten sind. Bevorzugte Untergruppen Z sind *xylo*-1,2,3,4-Tetrahydroxybutyl, *lyxo*-1,2,3,4-Tetrahydroxybutyl, *arabino-*1,2,3,4-Tetrahydroxybutyl, *threo*-1,2,3-Trihydroxypropyl, *erythro*-1,2,3-Trihydroxypropyl sowie *glycero*-1,2-Dihydroxyethyl. Die aus der D-Reihe der der Untergruppen Z zugrunde liegenden Kohlenhydrate ausgewählten Kohlenhydrate liefern meist die günstigsten Ergebnisse. Diastereomerenreine Untergruppen Z sind bevorzugt.

Besonders bevorzugte Verbindungen der Formel I sind ausgewählt aus der Gruppe bestehend aus
N-(*(2S)*-2-(3,4-Dichlorphenyl)-4-{4-[(2*S*,3*R*,4*R*)-2,3,4,5-tetrahydroxy-1-(3-thienyl)pentyl]-1-piperazinyl}butyl)-*N*-methylbenzamid;
N-(*(2S)*-2-(3,4-Dichlorphenyl)-4-{4[(2*S*)-2,3-dihydroxy-1-(2-furyl)propyl]-1-piperazinyl}-butyl)-N-methylbenzamid;
*(2S)*-2-(Acetyloxy)-3-{4-[*(3S)*-4-[benzoyl(methyl)amino]-3-(3,4-dichlorphenyl)butyl]-1-piperazinyl}-3-(2-furyl)propylacetat;
N-[*(2S)*-2-(3,4)-Dichlorphenyl)-4-(4-{2-furyl[*(4S)*-2-oxo-1,3-dioxolan-4-yl]methyl}-1-piperazinyl)butyl]-N-methyl-benzamid;
N-(*(2S)*-2-(3,4-Dichlorphenyl)-4-{4[(1*S*,2*R*)-2,3-dihydroxy-1-(3-thienyl)propyl]-1-piperazinyl}-butyl)-N-methylbenzamid;
N-(*(2S)*-2-(3,4-Dichlorphenyl)-4-{4[(2*S*)-2,3-dihydroxy-1-(3-furyl)propyl]-1-piperazinyl}-butyl)-N-methylbenzamid;
N-(*(2S)*-2-(3,4-Dichlorphenyl)-4-{4[(2*R*,3*R*,4*R*)-2,3,4,5-tetrahydroxy-1-(3-thienyl)pentyl]-1-piperazinyl}butyl)-N-methylbenzamid;
N-(*(2S)*-2-(3,4-Dichlorphenyl)-4-{4[(2*S*,3*R*,4*R*)*-*2,3,4,5-tetrahydroxy-1-(2-furyl)pentyl]-1-piperazinyl}-butyl)-N-methylbenzamid;
N-(*(2S)*-2-(3,4-Dichlorphenyl)-4-{4[(2*R*)-2,3-dihydroxy-1-(3-thienyl)propyl]-1-piperazinyl}-butyl)-N-methylbenzamid;
N-(*(2S)*-2-(3,4-Dichlorphenyl)-4-{4-[(2*R*,3*S*,4*S*)-2,3,4,5-tetrahydroxy-1-(3-thienyl)pentyl]-1-piperazinyl}-butyl)-N-methylbenzamid;
N-(*(2S)*-2-(3,4-Dichlorphenyl)-4-{4[2*S*,3*R*,4*R*)-2,3,4,5-tetrahydroxyacetyl-1-(3-thienyl)pentyl]-1-piperazinyl}-butyl)-N-methylbenzamid;
N-(*(2S)*-2-(3,4-Dichlorphenyl)-4-{4[(2*S*,3*R*)-2,3,4-trihydroxy-1-(3-thienyl)butyl]-1-piperazinyl}-butyl)-N-methylbenzamid und
N- (*(2S)*-2- (3, 4-Dichlorphenyl) -4-{2-furyl[(4*S*)-2-thioxo-1,3-dioxolan-4-yl]methyl}-1-piperazinyl)-butyl]-N-methylbenzamid.

Die Verbindungen der Formel I und deren Säureadditionssalze können hergestellt werden, indem man eine Verbindung der allgemeinen Formel II, 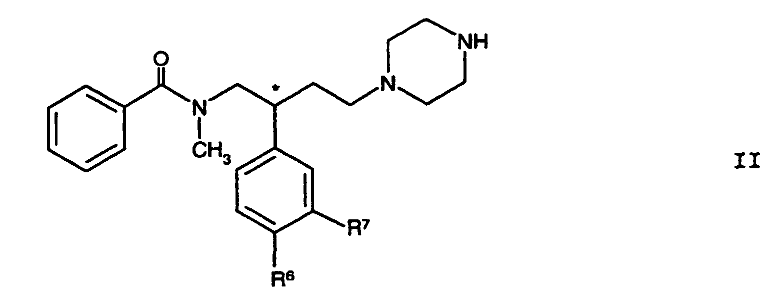 worin R⁶ und R⁷ obige Bedeutungen besitzen, mit einer Verbindung der allgemeinen Formel III,

A-B(OH)₂ **III**

worin A obige Bedeutung besitzt, und mit einer Verbindung der allgemeinen Formel IV, 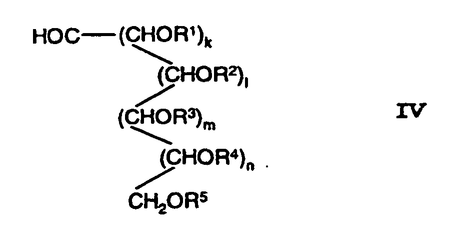 worin R¹, R², R³, R⁴, R⁵, k, l, m und n obige Bedeutungen besitzen, umsetzt, und eine erhaltene Verbindung der Formel I, worin mindestens ein Substituent, ausgewählt aus R¹, R², R³, R⁴ und R⁵, Wasserstoff bedeutet, anschließend gewünschtenfalls durch Umsetzung mit einer Verbindung der allgemeinen Formel VIII,

R⁸-COOH (VIII)

worin R⁸ die Bedeutung geradkettiges oder verzweigtes Alkyl mit 1 bis 3 Kohlenstoffatomen besitzt, in der Untergruppe Z acyliert oder eine erhaltene Verbindung der Formel I, worin mindestens zwei Substituenten, ausgewählt aus R¹, R², R³, R⁴ und R⁵ Wasserstoff bedeuten, anschließend gewünschtenfalls durch Umsetzung mit einem reaktiven Carbonyl- oder Thiocarbonyl-Syntheseäquivalent in der Untergruppe Z carbonyliert beziehungsweise thiocarbonyliert, oder eine erhaltene Verbindung der Formel I, worin mindestens zwei Substituenten, ausgewählt aus R¹, R², R³, R⁴ und R⁵, Wasserstoff bedeuten, durch Umsetzung mit einem Diniederalkylketon oder einem C₅₋₆-Cycloalkylketon in der Untergruppe Z zu einem über gegebenenfalls durch niederes Alkyl oder C₄₋₅-Alkylen substituiertes Methylen überbrückten 5-oder 6-Ring-Derivat umsetzt, und eine erhaltene Verbindung der Formel I gewünschtenfalls in ihr Säureadditionssalz überführt oder ein Säureadditionssalz in eine freie Verbindung der Formel I überführt.

Die Reaktion kann auf an sich bekannte Weise unter den Bedingungen einer Boron-Mannich-Reaktion (vgl. z. B. N.A. Petasis et al., Journal of the American Chemical Society 120 (1998) 11798-11799, WO 98/00398 oder WO 00/24510) durchgeführt werden. Demnach kann eine Verbindung der Formel II im Sinne einer Eintopfreaktion mit einer Boronsäure der Formel III und einem gegebenenfalls durch geeignete Schutzgruppen geschützten Kohlenhydrat der Formel IV in einem unter den Reaktionsbedingungen inerten Lösungsmittel umgesetzt werden. Geeignete Schutzgruppen für Kohlenhydrate sind an sich bekannt, beispielsweise aus J.A.W. McOmie, "Protective Groups in Organic Chemistry", Plenum Press, 1973, oder aus T.W. Green, P.G. Wuts, "Protective Groups in Organic Synthesis", Wiley and Sons, 1999. Als Lösungsmittel eignen sich dipolarprotische organische Lösungsmittel wie niedere Alkanole, beispielsweise geradkettige oder verzweigte C₁₋₄-Alkanole, vorzugsweise Ethanol, oder es eignen sich Gemische dieser vorgenannten Lösungsmittel mit Wasser oder mit dipolar-aprotischen Lösungsmitteln wie niederen Halogenalkanen, vorzugsweise Dichlormethan. Geeignete Reaktionstemperaturen liegen zwischen Raumtemperatur und dem Siedepunkt des Lösungsmittels oder des Lösungsmittelgemisches. Die Verbindungen der Formeln II, III und IV können vorzugsweise in dieser angegebenen Reihenfolge nacheinander zusammengefügt werden. Ebenso ist es auch möglich, eine Verbindung der Formel II zuerst mit einer Verbindung der Formel IV und anschließend mit einer Verbindung der Formel II zusammenzufügen. Das bei dieser Kopplungsreaktion neu entstehende, die Untergruppen A und Z tragende Chiralitätszentrum in Verbindungen der Formel I wird üblicherweise mit einem sehr hohen Maß an Diastereokontrolle als "anti"-Produkt gebildet.

Die Verbindungen der Formel I, welche in der Untergruppe Z mindestens eine freie Hydroxygruppe tragen, können gewünschtenfalls anschließend noch mit Verbindungen der Formel VIII umgesetzt werden, wodurch die freien Hydroxygruppen der Untergruppe Z acyliert werden. Üblicherweise findet unter diesen Umständen eine Peracylierung der freien Hydroxygruppen der Untergruppe Z statt. Als Acylierungsmittel können die Säuren der Formel VIII oder deren reaktionsfähige Derivate eingesetzt werden. Als reaktionsfähige Derivate kommen insbesondere Säureanhydride und Säurehalogenide in Frage. Die Acylierung kann in einem unter den Reaktionsbedingungen inerten organischen Lösungsmittel, vorzugsweise bei Temperaturen zwischen -20 °C und Raumtemperatur durchgeführt werden. Als Lösungsmittel eignen sich insbesondere aromatische Kohlenwasserstoffe wie Benzol oder Toluol, cyclische oder offenkettige Diniederalkylether wie Diethylether, Tetrahydrofuran (= THF) oder Dioxan, teilhalogenierte niedere Kohlenwasserstoffe wie Dichlormethan oder Gemische dieser Lösungsmittel. Sofern als Acylierungsmittel ein Säureanhydrid oder ein Säurehalogenid der Säuren der Formel VIII eingesetzt wird, kann die Acylierung zweckmäßigerweise in Gegenwart eines säurebindenden Reagenzes stattfinden. Als säurebindendes Reagenz eignen sich in dem Reaktionsgemisch lösliche nicht-nucleophile organische Basen wie Pyridin, Triethylamin oder 4-Dimethylaminopyridin. Im Überschuß eingesetzte organische Basen können gleichzeitig auch als Lösungsmittel eingesetzt werden.

Die Verbindungen der Formel I, welche in der Untergruppe Z mindestens zwei freie Hydroxygruppen tragen, können gewünschtenfalls nach ihrer vorstehend beschriebenen Herstellung anstelle einer Umsetzung mit Verbindungen der Formel VIII auch mit einem reaktiven Carbonyl- oder Thiocarbonyl-Syntheseäquivalent umgesetzt werden, wodurch die Untergruppe Z carbonyliert, beziehungsweise thiocarbonyliert werden kann. Die Umsetzung kann auf an sich bekannte Weise erfolgen. So kann eine Verbindung der Formel I in einem unter den Reaktionsbedingungen inerten organischen Lösungsmittel umgesetzt werden. Als reaktive Carbonyl-Syntheseäquivalente eignen sich beispielsweise Phosgen oder phosgenartig reagierende Substanzen wie Bis-(trichlormethyl)carbonat (= Triphosgen), Chlorameisensäure-trichlormethylester (= Diphosgen) oder insbesondere Carbonyldiimidazol. Als reaktives Thiocarbonyl-Synthese-äquivalent eignet sich vorzugsweise N,N'-Thiocarbonyldiimidazol. Zweckmäßig kann dem Reaktionsgemisch ein säurebindendes Reagenz zugesetzt werden. Als säurebindende Reagenzien eignen sich die vorstehend für die Umsetzung von Verbindungen der Formel I mit Verbindungen der Formel VIII angegebenen säurebindenden Reagenzien. Geeignete Reaktionstemperaturen liegen etwa zwischen -20 °C und Raumtemperatur.

Die Verbindungen der Formel I, welche in der Untergruppe Z mindestens zwei freie Hydroxygruppen tragen, können gewünschtenfalls nach ihrer vorstehend beschriebenen Herstellung anstelle einer Umsetzung mit Verbindungen der Formel VIII oder anstelle einer Umsetzung mit reaktiven Carbonyl- oder Thiocarbonyl-Syntheseäquivalenten auch mit einem Diniederalkylketon oder einem C₅₋₆-Cycloalkylketon in der Untergruppe Z zu einem über gegebenenfalls durch niederes Alkyl oder C₄₋₅-Alkylen substituiertes Methylen überbrückten 5-oder 6-Ring-Derivat umgesetzt werden. Als Diniederalkylketon eignet sich vorzugsweise Aceton. Als C₅₋₆-Cycloalkylketone eignen sich vorzugsweise Cyclopentanon und Cyclohexanon.

Sofern Verbindungen der Formel I hergestellt werden sollen, worin die in der Untergruppe Z enthaltenen Substituenten R¹, R², R³, R⁴ und/oder R⁵ andere Bedeutungen als Wasserstoff besitzen, wird vorzugsweise von Kohlenhydratverbindungen der Formel IV ausgegangen, welche zumindest in α-Stellung zu der Aldehydfunktion freie Hydroxygruppen enthalten. Günstig ist es, von Verbindungen der Formel IV auszugehen, worin R¹, R², R³, R⁴ und R⁵ Wasserstoff bedeuten. Die freien Hydroxygruppen können gewünschtenfalls anschließend in der vorstehend genannten Weise acyliert, carbonyliert, thiocarbonyliert oder mit einem geeigneten Keton umgesetzt werden.

Die Verbindungen der Formel II sind neue Verbindungen, welche sich in vorteilhafter Weise als Zwischenprodukte zur Herstellung neuer Wirkstoffe, beispielsweise zur Herstellung der Tachykininrezeptor-antagonistisch wirksamen Verbindungen der Formel I, eignen.

Die Verbindungen der Formel II können hergestellt werden, indem man eine Verbindung der allgemeinen Formel V, 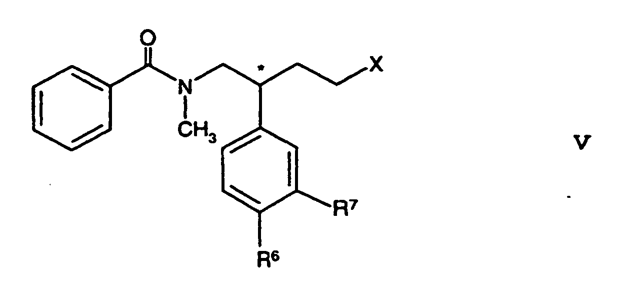 worin R⁶ und R⁷ obige Bedeutungen besitzen und X für Halogen, insbesondere für Iod steht, mit einem geschützten Piperazinderivat der allgemeinen Formel VI, 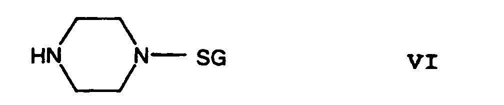 worin SG für eine abspaltbare Schutzgruppe, insbesondere für tert.-Butyloxycarbonyl, steht, umsetzt und nachfolgend die Schutzgruppe SG auf an sich bekannte Weise wieder abspaltet. Die Reaktion kann in einem unter den Reaktionsbedingungen inerten organischen Lösungsmittel wie einem aromatischen Kohlenwasserstoff, insbesondere Toluol, oder in einem cyclischen oder offenkettigen Diniederalkylether, insbesondere THF, oder vorzugsweise in einem Gemisch der vorgenannten Lösungsmittel und in Anwesenheit einer Base durchgeführt werden. Als Basen eignen sich nicht-nucleophile organische Stickstoffbasen wie tertiäre Niederalkylamine, beispielsweise Triethylamin. Geeignete Reaktionstemperaturen liegen zwischen 50° und 100 °C, vorzugsweise bei ca. 70° bis 90 °C.

Verbindungen der Formel V können hergestellt werden, indem man Verbindungen der allgemeinen Formel VII, 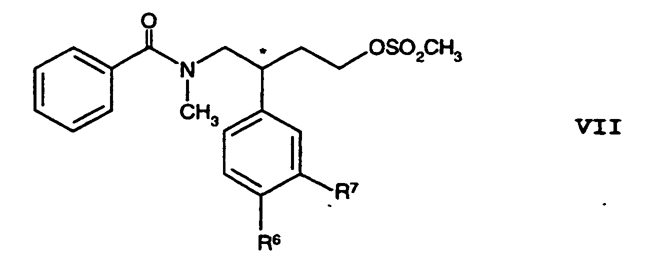 worin R⁶ und R⁷ obige Bedeutungen besitzen, auf an sich bekannte Weise mit einem Alkalimetallhalogenid der allgemeinen Formel MX, worin M für ein Alkalimetall, insbesondere Natrium, steht und X die obige Bedeutung besitzt und insbesondere für Iod steht, umsetzt. Die Verbindungen der Formel VII und deren stereoisomere Formen sind an sich bekannt, beispielsweise aus der EP 0 474 561 A1, und können nach den in dieser Schrift angegebenen oder nach dazu analogen Verfahren hergestellt werden.

Die Verbindungen der Formeln III, IV und VI sind an sich bekannt oder können vom Fachmann auf an sich bekannte Weise aus bekannten Verbindungen hergestellt werden. Bevorzugt eingesetzte Verbindungen der Formel IV umfassen D-Xylose, D-Lyxose, D-Arabinose, D-Threose, D-Erythrose sowie D- und L-Glycerinaldehyd.

Die Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in physiologisch verträgliche Säureadditionssalze überführt werden. Als physiologisch verträgliche Salze von Verbindungen der Formel I kommen deren übliche Salze mit anorganischen Säuren, beispielsweise Schwefelsäure, Phosphorsäuren oder Halogenwasserstoffsäuren, vorzugsweise Chlorwasserstoffsäure, oder mit organischen Säuren, beispielsweise niederen aliphatischen Mono-, Di- oder Tricarbonsäuren wie Maleinsäure, Fumarsäure, Milchsäure, Weinsäure, Zitronensäure, oder mit Sulfonsäuren, beispielsweise Niederalkansulfonsäuren wie Methansulfonsäure oder Trifluormethansulfonsäure, oder gegebenenfalls im Benzolring durch Halogen oder niederes Alkyl substituierte Benzolsulfonsäuren wie p-Toluolsulfonsäure, in Frage.

Die Verbindungen der Formel I enthalten in γ-Stellung zu dem Ringstickstoffatom in 4-Stellung des Piperazinringes ein asymmetrisches Kohlenstoffatom, nämlich das den durch R⁶ und R⁷ substituierten Phenylring tragende Kohlenstoffatom *C. Auf Grund dieses asymmetrischen Kohlenstoffatoms sowie auf Grund des die Untergruppen A und Z tragenden asymmetrischen Kohlenstoffatoms und gegebenenfalls auch auf Grund der in der Untergruppe Z enthaltenen asymmetrischen Kohlenstoffatome, können die Verbindungen der Formel I in mehreren stereoisomeren Formen vorliegen. Die vorliegende Erfindung umfaßt sowohl die Gemische optischer Isomere, als auch die isomerenreinen Verbindungen der Formel I. Bevorzugt sind Verbindungen der Formel I, worin das den durch R⁶ und R⁷ substituierten Phenylring tragende Kohlenstoffatom *C die S-Konfiguration aufweist. Falls bei der Synthese der Verbindungen der Formel I Gemische optischer Isomere der Ausgangsverbindung, beispielsweise der Verbindungen der Formel II oder der Verbindungen der Formel IV, eingesetzt werden, werden auch die Verbindungen der Formel I in Form von Gemischen optischer Isomere erhalten. Ausgehend von stereochemisch einheitlichen Formen der Ausgangsverbindung können auch stereochemisch einheitliche Verbindungen der Formel I erhalten werden. Die stereochemisch einheitlichen Verbindungen der Formel I können aus den Gemischen optischer Isomere in an sich bekannter Weise erhalten werden, z. B. durch chromatographische Trennung an chiralen Trennmaterialien oder durch Umsetzung mit geeigneten optisch aktiven Säuren, beispielsweise Weinsäure oder Campher-10-sulfonsäure, und anschließender Auftrennung in die optisch aktiven Antipoden durch fraktionierte Kristallisation der gewonnenen diastereomeren Salze.

Die Verbindungen der Formel I und deren Säureadditionssalze besitzen Tachykininrezeptor-antagonistische Eigenschaften und sind daher zur Behandlung von Krankheitszuständen bei größeren Säugetieren, insbesondere Menschen, geeignet, bei denen Tachykinine als Überträgerstoffe beteiligt sind. Die erfindungsgemäße Gruppe von Verbindungen zeichnet sich durch ein besonders günstiges Wirkprofil aus, welches gekennzeichnet ist durch eine hohe selektive Affinität zu NK-2-Rezeptoren. Weiterhin zeichnet sich die erfindungsgemäße Gruppe von Verbindungen durch eine gute Verträglichkeit auch über längere Applikationszeiträume und durch eine vergleichsweise gute orale Verfügbarkeit aus. Auf Grund ihres Wirkprofiles eignen sich die Verbindungen der Formel I insbesondere zur Hemmung von Vorgängen, an denen an NK-2-Rezeptoren bindende Tachykinine wie Neurokinin A beteiligt sind. Wegen der in vorteilhafter Weise auf den peripheren Bereich gerichteten Wirkung eignen sich die Verbindungen der Formel I insbesondere zur Behandlung und/oder Prophylaxe von funktionellen oder entzündlichen Störungen im gastrointestinalen Trakt von größeren Säugetieren, insbesondere Menschen, beiderlei Geschlechts, welche mit erhöhter Schmerzempfindlichkeit und/oder gestörter Stuhlpassage im Colonbereich verbunden sind. Zu den mit den erfindungsgemäßen Verbindungen behandelbaren funktionellen Störungen im gastrointestinalen Trakt gehören insbesondere die unter dem Namen "Irritable Bowel Syndrome" (= IBS) oder Reizdarmsyndrom bekannten Störungen der unteren Darmwege. Für die Diagnose von IBS typische Symptome werden beispielsweise beschrieben bei W.G. Thompson et al., Gastroenterology International 2 (1989) 92-95 oder bei W.G. Thompson et al., GUT 45/II (1999) II43-II47, und sind in der Fachwelt unter dem Begriff "Rome Criteria" allgemein bekannt. Zu den wesentlichen Symptomen von IBS zählen demnach Unterbauchschmerzen, die auf einer Übersensibilität des visceralen afferenten Nervensystems zu beruhen scheinen, und Anomalien des Stuhlganges wie Konstipation, Diarrhöe oder abwechselnd Konstipation und Diarrhöe. Weitere durch die erfindungsgemäße Gruppe von Verbindungen günstig beeinflußbare inflammatorisch bedingte Störungen im gastrointestinalen Trakt sind beispielsweise die meist unter dem Begriff "Inflammatory Bowel Disease" (= IBD) zusammengefaßten entzündlichen Störungen im Dünndarm- und Dickdarmbereich, beispielsweise Colitis ulcerosa oder Morbus Crohn. Aufgrund ihres Wirkmechanismus erscheinen die erfindungsgemäßen Verbindungen weiterhin geeignet zur Behandlung anderer Störungen, an denen Tachykinine und insbesondere Neurokinin A als Überträgerstoffe beteiligt sind. Zu diesen Störungen zählen beispielsweise neurogene Entzündungen, entzündliche Gelenkserkrankungen wie rheumatische Arthritis, asthmatische Beschwerden, allergische Störungen, Störungen der Immunregulation, Harnblasenentzündungen oder auch funktionelle Dyspepsie.

### Beschreibung der pharmakologischen Testmethoden

Die angegebenen Beispielsnummern der in den nachfolgend angegebenen pharmakologischen Tests als Testsubstanzen eingesetzten Verbindungen der Formel I beziehen sich auf die nachstehend beschriebenen Herstellungsbeispiele.

### 1. Bestimmung des Bindungsvermögens der Testsubstanzen an NK-2-Rezeptoren in vitro

Die Affinität der Testsubstanzen zu humanen NK-2-Rezeptoren wird in vitro gemessen. Bestimmt wird die Fähigkeit der Testsubstanzen, den als Referenzliganden verwendeten selektiven NK-2-Rezeptor-Antagonisten SR 48968 (= Saredutant) aus seiner entsprechenden Bindung zu verdrängen.

Die Rezeptorbindungsstudien werden mit radioaktiv markiertem [³H]-SR 48968 (Fa. Amersham) als Ligand durchgeführt. Für den Bindungsversuch werden verschiedene Proben einer Membranpräparation von CHO-Zellen (= Eizellen des chinesischen Hamsters, chinese hamster oocytes), die den humanen NK-2-Rezeptor (Herstellung s. N.P. Gerard et al., Journal of Biological Chemistry 265/33 (1990) 20455-20462) exprimieren, 90 Minuten (= min.) lang mit einer Lösung des markierten Liganden inkubiert, wobei die Inkubationsansätze keine Testsubstanz oder Zusätze unterschiedlicher Konzentrationen an Testsubstanz enthalten. Anschließend werden jeweils die in den Proben membrangebundenen Liganden von freien Liganden durch Filtration getrennt. Die im Filter verbleibende Fraktion wird mehrfach mit Pufferlösung gewaschen, bevor deren Radioaktivität mit einem Liquid-Scintillationszähler gemessen wird. Es wird als IC₅₀ der jeweiligen Testsubstanz diejenige Konzentration bestimmt, welche eine halbmaximale Verdrängung des gebundenen Referenzliganden bewirkt. Aus dem jeweiligen IC₅₀-Wert wird die Inhibitionskonstante (Ki-Wert) der Testsubstanz errechnet und als deren negativer logarithmierter Wert (pKi) angegeben.

Für die Verbindungen der Beispiele 1 bis 39 wurde die Affinität zu humanen NK-2-Rezeptoren jeweils durch mindestens dreimalige Messung der Testsubstanzen in Konzentrationsreihen von 10⁻⁶ bis 10⁻¹⁰ mol/L bestimmt. Sofern mehrere Messungen durchgeführt wurden, wurde jeweils deren Mittelwert angegeben. Alle vorgenannten Testsubstanzen zeigten in diesem Testmodell pKi-Werte von mindestens 7,0. Die Verbindungen der Beispiele 1 bis 27 und 39 zeigten pKi-Werte von mindestens 8,0. Die Verbindungen der Beispiele 1 bis 6 und 39 zeigten pKi-Werte von mindestens 9,0.

### 2. Bestimmung des funktionellen Antagonismus der Testsubstanzen an isoliertem Gewebe vom Meerschweinchen in vitro

Die NK-2-Rezeptor antagonisierende Wirkung der Testsubstanzen wird an isolierten, in einer sauerstoffgesättigten Nährlösung gehaltenen Gallenblasenpräparaten von Pirbright-White Meerschweinchen bestimmt. Hierzu werden die Präparate einerseits an Organhaltern in der Nährlösung und andererseits mit einem Faden an einem Kraftmesser fixiert.

In diesem Test werden die in den Gallenblasenpräparaten vorliegenden NK-2-Rezeptoren mit dem natürlichen NK-2-Rezeptor-Agonisten Neurokinin A (= NKA; 0,1 µmol/L) stimuliert und die dadurch verursachten Kontraktionen der Präparate werden als Kontraktionskraft in mN (= Vorwert) gemessen. Anschließend wird NKA mit NKA-freier Lösung aus den Präparaten ausgespült und die Testsubstanzen werden in der Konzentration 10⁻⁷ mol/L hinzugefügt. Nach zweistündiger Inkubation der Präparate mit den Testsubstanzen werden die dann durch erneute NKA-Zugabe noch hervorgerufenen Kontraktionen der Präparate wiederum gemessen und die Ergebnisse werden als Prozentwerte, bezogen auf die anfangs gemessenen, durch alleinige NKA-Zugabe verursachten Kontraktionen angegeben. Die Konzentration der Testsubstanzen wird in den Folgeexperimenten in Abhängigkeit vom Resultat in logarithmischen Ganz- bzw. Halbschritten iterativ erhöht, bis mindestens eine Konzentration über bzw. unter 50 % Kontraktionshemmung ermittelt ist (bis maximal 10⁻⁵ mol/L). Für jede Konzentration wird der Mittelwert der Kontraktionshemmung aus 2 bis 4 Präparaten errechnet. Als Kenngrösse wird jeweils die Konzentration halbmaximaler Hemmung (IC₅₀) pro Testsubstanz errechnet. Angegeben wird als pIC₅₀ in [mol/L] jeweils der logarithmierte Wert der IC₅₀ pro Testsubstanz. In diesem Testmodell zeigten die in der folgenden Tabelle 1 aufgeführten Testsubstanzen die nachfolgend angegebenen pIC₅₀-Werte.

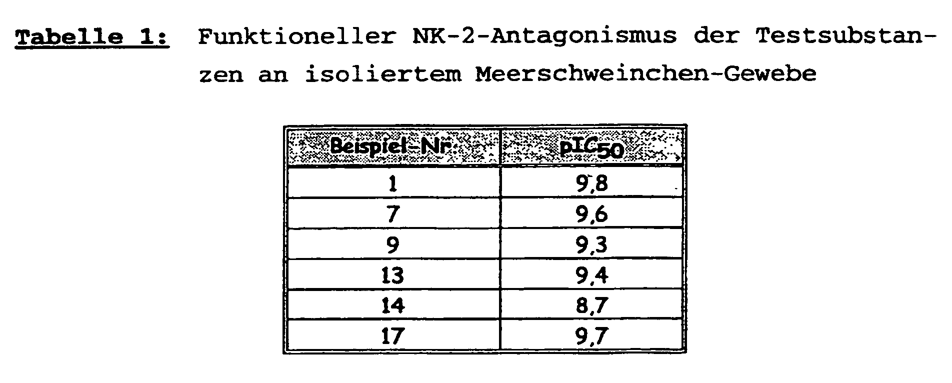

### 3. Bestimmung der NK-2-Rezeptor-antagonistischen Wirksamkeit der Testsubstanzen in vivo

Die NK-2- und NK-1-antagonistischen Aktivitäten der Testsubstanzen werden im narkotisierten Meerschweinchen jeweils nach intravenöser (= i.v.) und oraler (= p.o.) Gabe in vivo untersucht. Mit dem vorliegenden Testmodell ist es möglich, sowohl NK-2-antagonistische Effekte in drei verschiedenen Organsystemen (Atemwege, Colon und Kreislauf) als auch NK-1-antagonistische Effekte (schneller Blutdruckabfall) in einem Tier gleichzeitig zu erfassen.

Pirbright-White-Meerschweinchen von 500-700 g Körpergewicht werden mit Ketamin/Xylazin (67/13 mg/kg subkutan, initiale Dosis, weitere Gaben nach Bedarf) narkotisiert. Die Tiere werden mit einem intravenösen Katheter zur Substanzgabe und einem intraarteriellen Katheter zur Messung des Blutdruckes versehen. Über eine Tracheakanüle werden die Tiere künstlich beatmet und der Atemdruck wird über einen Druckaufnehmer registriert. Zur manometrischen Registrierung der Colonmotilität über einen Druckaufnehmer wird den Tieren ein Ballon in das distale Colon eingeführt. Blutdruck, Herzfrequenz, Atemdruck und Colondruck werden bei jedem Tier kontinuierlich gemessen und auf einen Schreiber sowie über ein digitales Datenerfassungssystem aufgezeichnet. Als Testreiz zur Stimulation der NK-1- und der NK-2-Rezeptoren wird Neurokinin A (= NKA; 200 pmol/ Tier) als Bolus i.v. verabreicht. Eine solche Gabe von NKA führt zu einer starken Erhöhung des Atemdrucks (Bronchokonstriktion) und des Colondrucks, sowie zu einem biphasischen Abfall des Blutdruckes. Die erste Phase der Hypotension (= Phase der maximalen Hypotension innerhalb der ersten Minute nach NKA-Gabe) ist über NK-1-Rezeptoren vermittelt, da sie durch spezifische NK-1-Rezeptor-Antagonisten vollständig blockiert werden kann. Die zweite Phase verzögerter Hypotension (= Phase der maximalen Hypotension nach 2-5 Min.) ist dagegen über NK-2-Rezeptoren vermittelt, da sie durch spezifische NK-2-Rezeptor-Antagonisten blockiert werden kann. Als charakteristische Größen für die einzelnen Meßparameter Bronchokonstriktion, Colondruck sowie NK-1- bzw. NK-2-vermittelte Blutdruckänderung werden die Dosen der Testsubstanzen als ED₅₀-Werte angegeben, die jeweils zu einer auf 50 % des Ausgangswertes reduzierten Antwort auf den Teststimulus NKA führen.

Die antagonistischen Effekte der Testsubstanzen wurden zuerst in kumulativer Form untersucht, wobei der Zeitpunkt des NKA-Teststimulus 1 min. nach Ende der Applikation der jeweiligen Dosen der Testsubstanzen lag. Diese aus kumulativen Dosis-Wirkungskurven gewonnenen ED₅₀-Werte sind in der nachfolgenden Tabelle 2 (Zeile 1) aufgetragen. Um zusätzlich den Zeitverlauf der antagonistischen Effekte der Testsubstanzen zu erfassen, wurde die Wirkung des NKA-Teststimulus zu unterschiedlichen Zeitpunkten (1, 30, 60, 90, 120, 150 und 180 Min.) nach Applikation der Testsubstanzen bestimmt. Die antagonistischen Effekte der Testsubstanzen wurden dann als Fläche unter der Kurve ("area under the curve", "AUC") über den Untersuchungszeitraum nach der Applikation der Testsubstanzen (i.v.: 120 min. nach Applikation; p.o.: 180 min. nach Applikation) bestimmt und die daraus gewonnenen ED₅₀-Werte wurden in der nachfolgenden Tabelle 2 (Zeilen 2 und 3) aufgetragen.

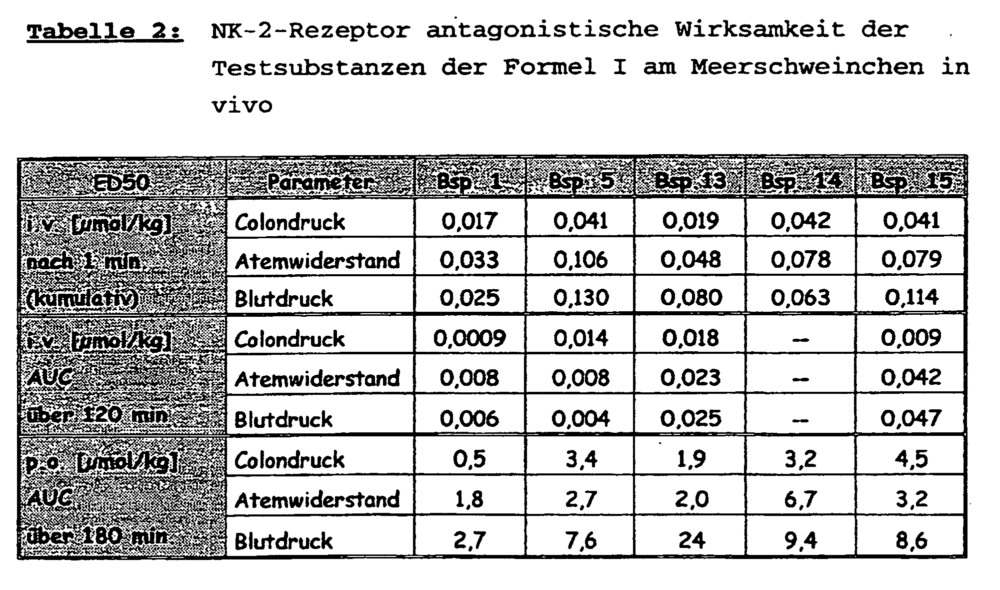

Die in der vorstehenden Tabelle 2 aufgetragenen Meßwerte zeigen u. a., daß die Substanzen der Beispiele 1, 5, 13, 14 und 15 nach kumulativer Applikation i.v. (Erfassung des Antagonismus 1 min. nach Ende der Testsubstanzgabe) eine ausgeprägte NK-2-Rezeptor-antagonistische Aktivität der Colonmotiltät, des späten Blutdruckabfalls und des Atemwiderstandes verursachten.

Die in der vorstehenden Tabelle 2 aufgetragenen Meßwerte zeigen auch, daß die vorgenannten Substanzen, insbesondere die Substanz des Beispieles 1, eine effektivere Hemmung der NK-2-Mechanismen am Colon (Colonpräferenz) im Vergleich zur Hemmung der bronchokonstriktorischen bzw. hypotensiven NK-2-Effekte verursachten. Die erfindungsgemäßen Verbindungen, insbesondere die Substanz des Beispieles 1, zeichnen sich weiterhin durch eine langsam eintretende und langanhaltende Wirkung aus.

Eine NK-1-Rezeptor-antagonistische Wirkung konnte bei den verwendeten Dosen in vivo für keine der untersuchten Testsubstanzen beobachtet werden.

Die Verbindungen der Formel I können in üblichen pharmazeutischen Zubereitungen verabreicht werden. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größeren Säugetieren jedoch Arzneiformen mit einem Wirkstoffgehalt von 0,2 bis 200 mg, insbesondere 1 bis 50 mg Wirkstoff pro Einzeldosis. Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese Präparate können pharmazeutisch übliche anorganische und/oder organische Trägerstoffe, wie z. B. Talkum, Milchzucker oder Stärke, neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die nachfolgend angeführten Beispiele sollen die Erfindung näher erläutern, ohne sie in ihrem Umfang zu beschränken.

### Beispiel 1:

### N-((2S)-2-(3,4-Dichlorphenyl)-4-{4-[(2S,3R,4R)-2,3,4,5-tetrahydroxy-1-(3-thienyl)pentyl]-1-piperazinyl}butyl)-N-methylbenzamid

A) 45,0 g N-[(2*S*)-2-(3,4-Dichlorphenyl)-4-methansulfonyloxyl-N-methylbenzamid wurden unter Schutzgasatmosphäre in 550 ml Aceton gelöst. Zu dieser Vorlage gab man 84,6 g NaI hinzu und rührte die erhaltene Suspension 20 h lang bei Raumtemperatur. Man dampfte das Lösungsmittel im Vakuum weitgehend ein und nahm den verbleibenden Rückstand in 650 ml Methyl-tert.-butylether (= MTBE) und 500 ml Wasser auf. Nach Zugabe von 120 g Na₂S₂O₄ wurde die wäßrige Phase abgetrennt und die verbleibende organische Phase wurde viermal mit je 100 ml gesättigter wäßriger Kochsalzlösung gewaschen. Man trocknete die organische Phase über Natriumsulfat und dampfte das Lösungsmittel im Vakuum ein. Trocknung des verbleibenden Rückstandes im Vakuum lieferte 45,9 g N-[(2*S*)-2-(3,4-Dichlorphenyl)-4-jodbutyl]-N-methylbenzamid] als glasige Verbindung, welche ohne weitere Reinigung direkt für weitere Umsetzungen verwendet wurde.
B) 15,38 g N-tert.-Butyloxycarbonyl-Piperazin wurden bei Raumtemperatur unter Schutzgasatmosphäre in 200 ml Toluol gelöst und mit 32 ml Triethylamin versetzt. Die entstandene Lösung wurde auf 84 °C erhitzt. Zu dieser Vorlage tropfte man langsam 45,9 g des vorstehend erhaltenen Jodids, gelöst in einem Gemisch aus 100 ml THF und 200 ml Toluol, hinzu. Das so erhaltene Reaktionsgemisch wurde 5 h lang auf 80 bis 85 °C erhitzt und anschließend noch 8 h lang bei Raumtemperatur gerührt. Das Lösungsmittelgemisch wurde im Vakuum weitgehend eingedampft und der verbleibende Rückstand wurde in 600 ml Essigsäureethylester (= EE) aufgenommen. Nach Abtrennung des ausgefallenen Niederschlages wurde die organische Phase der Reihe nach zweimal mit je 100 ml Wasser und 100 ml 15%iger wäßriger Weinsäurelösung gewaschen. Anschließend gab man zu der organischen Phase 8,0 g NaOH zu und wusch erneut zweimal mit je 200 ml Wasser. Trocknung der organischen Phase über Natriumsulfat und Eindampfen des Lösungsmittels im Vakuum lieferte 44,9 g tert.-Butyl-4-[(3S)-4-[benzoyl(methyl)amino]-3-(3,4-dichlorphenyl)-butyl]-1-piperazincarboxylat als ölige Verbindung, welche ohne weitere Reinigung direkt für weitere Umsetzungen verwendet wurde.
C) 44,5 g der vorstehend erhaltenen Piperazincarboxylat-Verbindung wurden bei Raumtemperatur in 600 ml Methanol gelöst, mit 150 ml 6 N HCl versetzt und 60 h lang gerührt. Anschließend gab man 500 ml Wasser zu und dampfte die Methanol-Phase im Vakuum weitgehend ein. Die verbliebene wäßrige Phase wurde viermal mit je 100 ml EE und viermal mit je 100 ml MTBE extrahiert. Anschließend gab man zu der wäßrigen Phase eine Lösung von 36,0 g NaOH in 200 ml Wasser zu und extrahierte die nunmehr alkalische wäßrige Phase noch zweimal mit je 350 ml EE. Die vereinigten organischen Phasen wurden mit 100 ml Wasser gewaschen, über Natriumsulfat getrocknet und im Vakuum eingedampft. Trocknung des Rückstandes lieferte 25,8 g N-[(2*S*)-2-(3,4-Dichlorphenyl)-4-(piperazinyl)-butyl]-N-methylbenzamid als gelbliches, festes Öl, welches ohne weitere Reinigung direkt für weitere Umsetzungen verwendet wurde.
D) 25,0 g der vorstehend erhaltenen entschützten Piperazin-verbindung wurden unter Schutzgasatmosphäre bei 30 °C in 250 ml Ethanol gelöst. Nach Erwärmen dieser Vorlage auf 50 bis 60 °C gab man erst 10,0 g Thiophen-3-boronsäure und anschließend 8,93 g D-Xylose zu. Es wurde 15 h lang unter Rückflußkühlung zum Sieden erhitzt und anschließend noch 8 h lang bei Raumtemperatur gerührt. Man gab 500 ml Wasser hinzu und dampfte das Lösungsmittelgemisch weitgehend im Vakuum ein. Zu dem verbliebenden Rückstand gab man 20 ml 6 N HCl hinzu und wusch der Reihe nach erst einmal mit 200 ml EE, dann sechsmal mit je 100 ml EE. Man stellte die wäßrige Phase durch Zugabe einer entsprechenden Menge an 4 N NaOH auf pH 9 bis 10 ein und extrahierte dann mit 600 ml Dichlormethan. Die organische Phase wurde abgetrennt, über Natriumsulfat getrocknet und schließlich im Vakuum eingedampft. Man erhielt 32,0 g der Titelverbindung als amorphen Feststoff, optischer Drehwert [α]_{D}²⁰= -14,8 (c = 1 in Methanol); ¹H-NMR (d₆-DMSO, 90°C): 3,81 (d,1H); 4,15(dd, 1H); 3,79 (dd, 1H); 3,66 (ddd,1H); 3,48(m, 2H); 7,12 (dd, 1H); 7,19 (d, 1H); 7,38(dd, 1H).

### Beispiel 2:

### N-((2S)-2-(3,4-Dichlorphenyl)-4-{4[(2S)-2,3-dihydroxy-1-(2-furyl)propyl]-1-piperazinyl}-butyl)-N-methylbenzamid

2,08 g N-[(2*S*)-2-(3,4-Dichlorphenyl)-4-(piperazinyl)butyl]-N-methylbenzamid (Herstellung s. o. unter 1C) wurden in 100 ml Ethanol gelöst und auf ca. 50 °C erwärmt. Zu dieser Vorlage wurden 740 mg 2-Furanboronsäure und 550 mg einer 80%igen Lösung von D-Glycerinaldehyd in Wasser zugegeben. Die entstandene Lösung wurde 10 h lang am Rückfluß zum Sieden erhitzt. Anschließend dampfte man überschüssiges Lösungsmittel im Vakuum ein. Reinigung des Rückstandes durch Säulenchromatographie an Kieselgel (Laufmittel: Dichlormethan/EtOH/NH₄OH 87/11/2) lieferte 2,1 g der Titelverbindung als beigen Schaum, optischer Drehwert [α]_{D}²⁰ = -6,1° (c = 1 in Methanol); ¹H-NMR (CDCl₃, RT): 3,65 (d, 1H); 4,23 (ddd, 1H); 3,72 (dd,1H); 3,78(dd, 1H); 6,27 (d, 1H); 6,37 (1H); 7,42(1H).

### Beispiel 3:

### (2S)-2-(Acetyloxy)-3-{4-[(3S)-4-[benzoyl(methyl)amino]-3-(3,4-dichlorphenyl)butyl]-1-piperazinyl}-3-(2-furyl)propylacetat

Zu einer Lösung von 400 mg N-((2*S*)-2-(3,4-Dichlorphenyl)-4-{4-[(2*S*)-2,3-dihydroxy-1-(2-furanyl)propyl]-1-piperazinyl} butyl)-N-methylbenzamid (Herstellung s. Beispiel 2) in 10 ml Pyridin wurden 1,5 g Essigsäureanhydrid bei Raumtemperatur zugegeben. Man rührte das Reaktionsgemisch 72 h lang und goß es anschließend in eine Lösung von 2,2 g Na₂CO₃ in 40 ml Wasser. Die organische Phase wurde mit 60 ml Toluol extrahiert und die Toluolphase wurde je dreimal mit 30 ml Wasser und je zweimal mit gesättigter wäßriger Kochsalzlösung gewaschen. Man trocknete die vereinigten organischen Phasen über Natriumsulfat und dampfte das Lösungsmittel im Vakuum ein. Man erhielt 463 mg der Titelverbindung als gelblichen Schaum, [α]_{D}²⁰ = + 9,6° (c = 1 in Methanol); ¹H-NMR (CDCl₃, RT): 3,81 (d, 1H); 5,62(ddd, 1H); 4,58(dd, 1H); 4,21(dd, 1H); 1,87 (s, 3H); 2,05 (s, 3H); 6,16 (d, 1H); 6,31(1H); 7,35(1H).

### Beispiel 4:

### N-[(2S)-2-(3,4)-Dichlorphenyl)-4-(4-{2-furyl[(4S)-2-oxo-1,3-dioxolan-4-yl]methyl}-1-piperazinyl)butyl]-N-methyl-benzamid

Zu einer Lösung von 576 mg N-(*(2S)*-2-(3,4-Dichlorphenyl)-4-{4-[(2*S*)-2,3-dihydroxy-1-(2-furyl)propyl]-1-piperazinyl}-butyl)-N-methylbenzamid (Herstellung s. Beispiel 2) in 30 ml trockenem Dichlormethan wurden bei Raumtemperatur 124 mg 4-Dimethylaminopyridin (= DMAP) und 410 mg N-Carbonyldiimidazol zugegeben. Man rührte das Reaktionsgemisch 15 h lang bei Raumtemperatur und gab anschließend 3,7 g Kieselgel zu. Man rührte die entstanden Suspension noch 1 h lang, trennte die flüssige Phase durch Vakuumfiltration ab und engte das Filtrat im Vakuum ein. Den verbleibenden Rückstand nahm man in 50 ml EE auf und wusch die organische Phase fünfmal mit je 10 ml einer 50:50 (v/v) Mischung von 5%iger wäßriger KH₂PO₄-Lösung und 1%iger wäßriger K₂HPO₄-Lösung. Die organische Phase wurde über Natriumsulfat getrocknet und das Lösungsmittel wurde anschließend im Vakuum eingedampft. Man erhielt 460 mg der Titelverbindung als weißen Schaum, ¹H-NMR (CDCl₃, RT): 3,73(d, 1H); 5,07(ddd, 1H); 4,56(dd, 1H); 4,48(dd, 1H); 6.3(d, 1H); 6,378(1H); 7,41(1H).

Die erhaltene Titelverbindung wurde in 4 ml Methanol gelöst und man gab 0,31 ml einer 1,6 M HCl in Isopropanol zu. Man erhielt das Dihydrochlorid der Titelverbindung, [α]_{D}²⁰ = -28° (c = 1 in Methanol).

Nach der in den vorstehenden Beispielen beschriebenen oder nach hierzu analogen Verfahren können auch die in der nachfolgenden Tabelle 3 angegebenen Verbindungen der Formel I hergestellt werden.

Die in der nachfolgenden Tabelle 3 angegebenen Verbindungen der Beispiele 5 bis 38 wurden nach einem automatisierten Herstellungsverfahren hergestellt. Hierzu wurden pro Ansatz jeweils 200 µl einer 0,25 N wäßrigen Vorratslösung der entsprechenden Kohlenhydratverbindung der Formel IV in einem Mikroreaktionsgefäß abgemessen und zur weitgehenden Entfernung des Wassers im Vakuum eingedampft. Der Rückstand wurde in 200 µl Ethanol aufgenommen. Zu dieser Vorlage wurden jeweils 200 µl einer 0,25 mol/L ethanolischen Vorratslösung von racemischen bzw. enantiomerenreinen (vgl. jeweils die entsprechende Angabe in Tabelle 3) N-[2-(3,4-Dichlorphenyl)-4-(1-piperazinyl)butyl]-N-methylbenzamid der Formel II sowie 200 µl einer 0,25 N ethanolischen Vorratslösung der entsprechenden Boronsäure (= Dihydroxyboran-Verbindung) der Formel III hinzugegeben. Das Reaktionsgemisch wurde zuerst 2 h lang auf 80 °C erhitzt und dann auf Raumtemperatur abgekühlt und mit 1 ml Ethanol versetzt. Anschließend fügte man 100 mg basisches Amberjet®-Ionenaustauscherharz hinzu und schüttelte das Reaktionsgefäß 2 h lang. Man filtrierte vom Ionenaustauscher ab, wusch zweimal mit je 500 µl Ethanol nach und dampfte das Lösungsmittel im Vakuum zur Trockene ein. Aus dem Rückstand wurde ohne weitere Aufreinigung jeweils eine Probe für die Hochleistungs-Flüssigkeitschromatographie (= HPLC) und für die automatische Massenspektroskopie zur Bestimmung der Reinheit bzw. zur Strukturbestätigung entnommen.

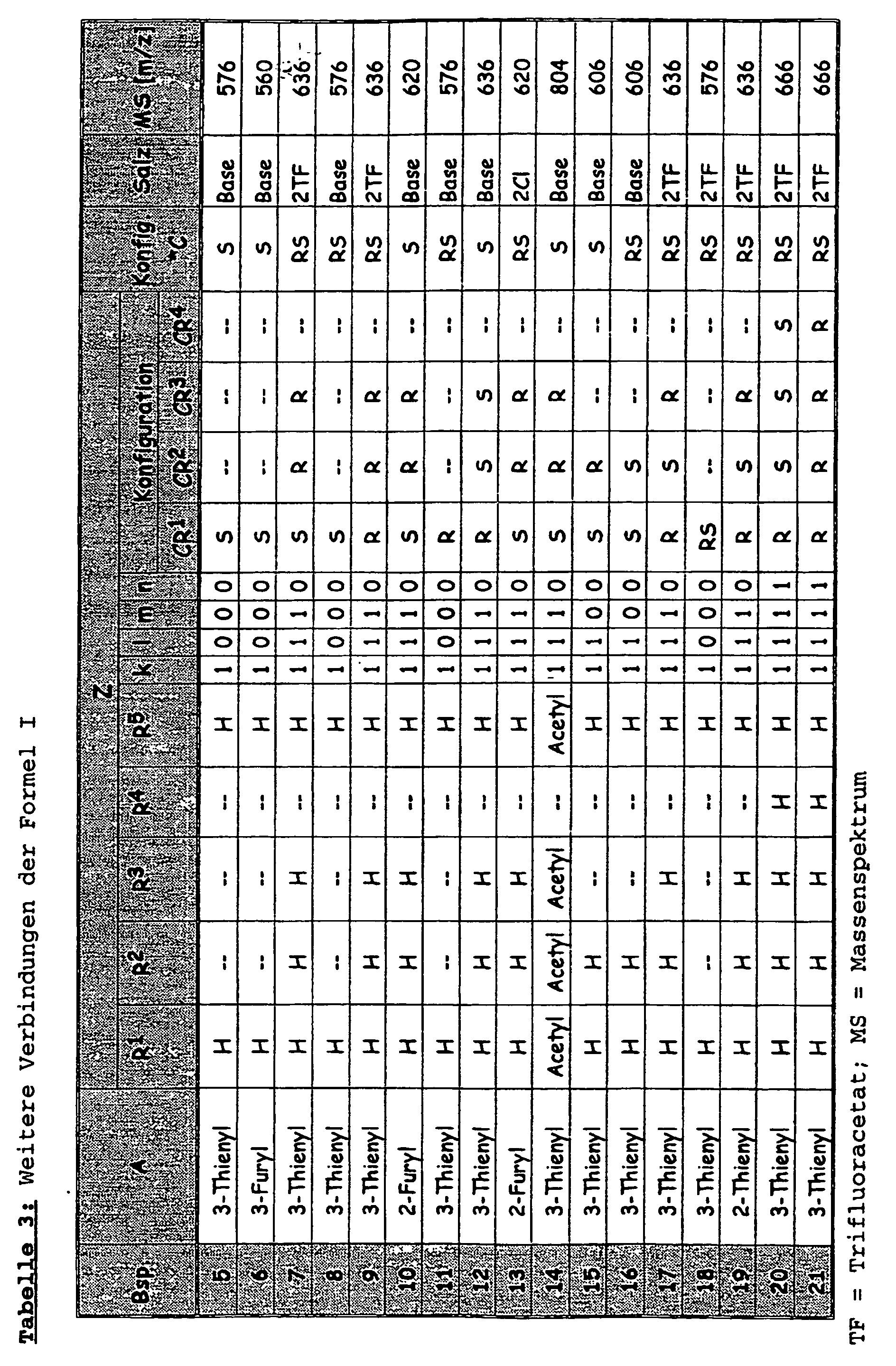

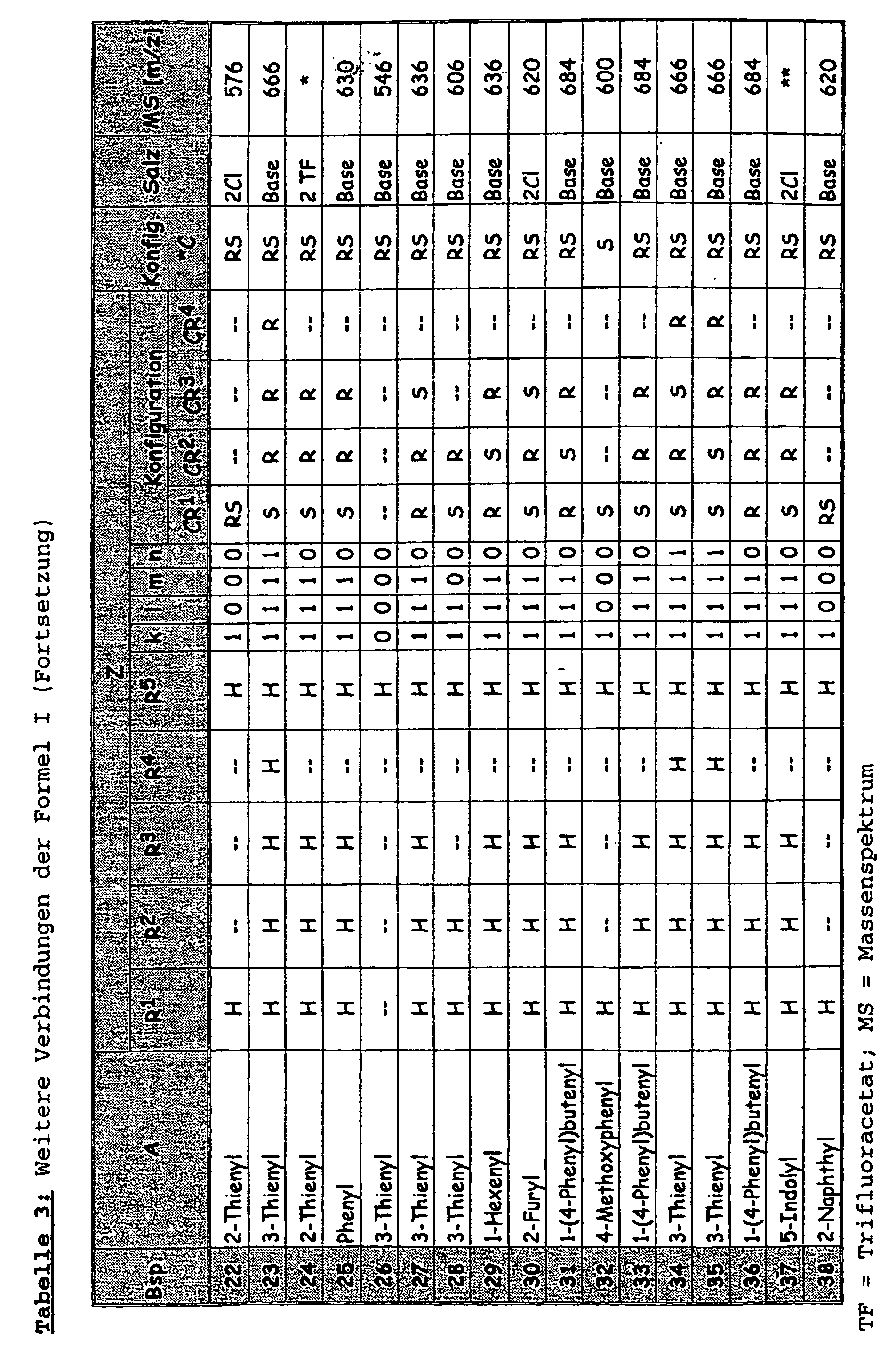

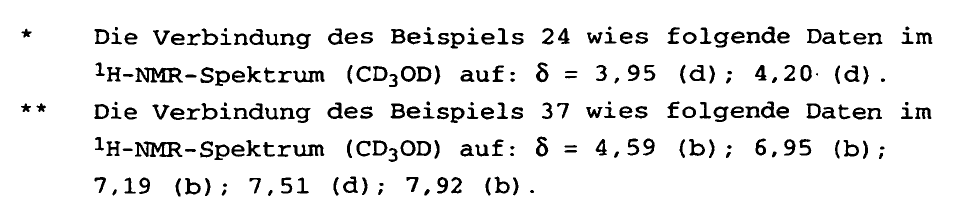

### Beispiel 39:

### N-((2S)-2-(3,4-Dichlorphenyl)-4-{2-furyl[(4S)-2-thioxo-1,3-dioxolan-4-yl]methyl}-1-piperazinyl)-butyl]-N-methylbenzamid

Zu einer Lösung von 303 mg N-(*(2S)*-2-(3,4-Dichlorphenyl)-4-{4-[(2*S*)-2,3-dihydroxy-1-(2-furyl)propyl]-1-piperazinyl}-butyl)-N-methylbenzamid (Herstellung s. Beispiel 2) in 10 ml trockenem Dichlormethan wurden bei Raumtemperatur 240 mg N,N'-Thiocarbonyldiimidazol zugegeben. Man rührte das Reaktionsgemisch 20 h lang bei Raumtemperatur und engte anschließend im Wasserstrahlpumpenvakuum ein. Den verbleibenden Rückstand nahm man in 50 ml EE auf und wusch die organische Phase fünfmal mit Wasser. Die organische Phase wurde über Natriumsulfat getrocknet und das Lösungsmittel wurde anschließend im Vakuum (zuerst Wasserstrahlpumpe, dann Ölpumpe) eingedampft. Säulenchromatographie des erhaltenen gelblichen Schaumes (stationäre Phase: Kieselgel; mobile Phase: n-Hexan/Aceton 1:1) lieferte 118 mg der amorphen Titelverbindung, ¹H-NMR (CDCl₃, RT): 3,79 (d, 1H); 5,10 (ddd, 1 H); 6,27 (1H); 6,36 (1H).

### Beispiel I:

### N-((2S)-2-(3,4-Dichlorphenyl)-4-{4-[(2S,3R,4R)-2,3,4,5-tetrahydroxy-1-(3-thienyl)pentyl]-1-piperazinyl}butyl)-N-methylbenzamid enthaltende Kapseln:

Es werden Kapseln in folgender Zusammensetzung pro Kapsel hergestellt:

| | |
|---|---|
| N- (2*S*)-2-(3,4-Dichlorphenyl)-4-{4-[(2*S*,3*R*,4*R*)- | |
| 2,3,4,5-tetrahydroxy-1-(3-thienyl)pentyl]-1-pipera- | |
| zinyl}butyl)-*N*-methylbenzamid | 20 mg |
| Maisstärke | 60 mg |
| Milchzucker | 300 mg |
| Ethylacetat | q.s. |

Der Wirkstoff, die Maisstärke und der Milchzucker werden unter Zuhilfenahme von EE zu einer homogenen pastösen Mischung verarbeitet. Die Paste wird zerkleinert und das entstehende Granulat wird auf ein geeignetes Blech gebracht und zur Entfernung des Lösungsmittels bei 45 °C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann in 400 mg fassende Kapseln (= Kapselgröße 0) abgefüllt.

## Patentansprüche

1. Verbindungen der allgemeinen Formel I 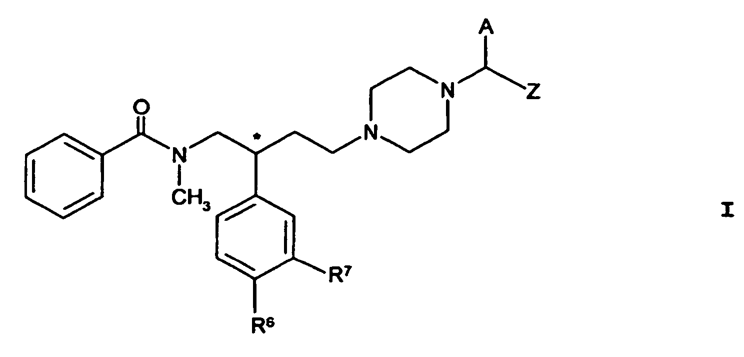 worin
A Naphthyl, gegebenenfalls durch Hydroxy substituiertes Phenyl, mono- oder bicyclisches Heteroaryl oder gegebenenfalls durch Phenyl substituiertes C₃₋₆-Alkenyl bedeutet,
Z für eine Untergruppe der allgemeinen Formel 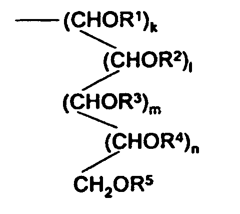 steht, worin
R¹ Wasserstoff oder C₂₋₄-Alkanoyl bedeutet, oder gemeinsam mit einem anderen Substituenten, ausgewählt aus der Gruppe bestehend aus R², R³, R⁴ und R⁵, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
R² Wasserstoff oder C₂₋₄-Alkanoyl bedeutet, oder gemeinsam mit einem anderen Substituenten, ausgewählt aus der Gruppe bestehend aus R¹, R³, R⁴ und R⁵, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
R³ Wasserstoff oder C₂₋₄-Alkanoyl bedeutet, oder gemeinsam mit einem anderen Substituenten, ausgewählt aus der Gruppe bestehend aus R¹, R², R⁴ und R⁵, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
R⁴ Wasserstoff oder C₂₋₄-Alkanoyl bedeutet, oder gemeinsam mit einem anderen Substituenten, ausgewählt aus der Gruppe bestehend aus R¹, R², R³ und R⁵, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
R⁵ Wasserstoff oder C₂₋₄-Alkanoyl bedeutet, oder gemeinsam mit einem anderen Substituenten ausgewählt aus der Gruppe bestehend aus R¹, R², R³ und R⁴, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
k 0 oder 1 bedeutet,
l 0 oder 1 bedeutet,
m 0 oder 1 bedeutet und
n 0 oder 1 bedeutet,
R⁶ Halogen oder Wasserstoff bedeutet und
R⁷ Halogen oder Wasserstoff bedeutet,
sowie physiologisch verträgliche Säureadditionssalze von Verbindungen der Formel I.

2. Verbindungen nach Anspruch 1, worin A Thiophen oder Furan bedeutet.

3. Verbindungen nach Anspruch 1, worin k 1 bedeutet und n 0 bedeutet.

4. Verbindungen nach Anspruch 1, worin R⁶ und R⁷ jeweils für Chlor stehen.

5. Verbindungen nach Anspruch 1, worin das Chiralitätszentrum *C die S-Konfiguration aufweist.

6. Verbindungen der Formel I gemäß einem der vorstehenden Ansprüche, welche ausgewählt sind aus der Gruppe bestehend aus
*N*-(*(2S)*-2-(3,4-Dichlorphenyl)-4-{4-[(2*S*,3*R*,4*R*)-2,3,4,5-tetrahydroxy-1- (3-thienyl)pentyl]-1-piperazinyl}butyl)*-N*-methylbenzamid;
N-(*(2S)*-2-(3,4-Dichlorphenyl)-4-{4[(2*S*)-2,3-dihydroxy-1-(2-furyl)propyl]-1-piperazinyl}-butyl)-N-methylbenzamid ;
*(2S)*-2-(Acetyloxy)-3-{4-[*(3S)*-4-[benzoyl(methyl)amino]-3-(3,4-dichlorphenyl)butyl]-1-piperazinyl}-3-(2-furyl)propylacetat;
N-[*(2S)*-2-(3,4)-Dichlorphenyl)-4-(4-{2-furyl[*(4S)*-2-oxo-1,3-dioxolan-4-yl]methyl}-1-piperazinyl)butyl]-N-methyl-benzamid;
N-(*(2S)*2-(3,4-Dichlorphenyl)-4-{4[(1*S*,2*R*)-2,3-dihydroxy-1-(3-thienyl)propyl]-1-piperazinyl}-butyl)-N-methylbenzamid;
N-(*(2S)*2-(3,4-Dichlorphenyl)-4-{4[(2*S*)-2,3-dihydroxy-1-(3-furyl)propyl]-1-piperazinyl}-butyl)-N-methylbenzamid;
N-(*(2S)*-2-(3,4-Dichlorphenyl)-4-{4[(2*R*,3*R*,4*R*)-2,3,4,5-tetrahydroxy-1-(3-thienyl)pentyl]-1-piperazinyl}butyl)-N-methylbenzamid;
N-(*(2S)*-2-(3,4-Dichlorphenyl)-4-{4[(2*S*,3*R*,4*R*)-2,3,4,5-tetrahydroxy-1-(2-furyl)pentyl]-1-piperazinyl}-butyl)-N-methylbenzamid;
N-(*(2S)*-2-(3,4-Dichlorphenyl)-4-{4[(2*R*)-2,3-dihydroxy-1-(3-thienyl)propyl]-1-piperazinyl}-butyl)-N-methylbenzamid;
N-(*(2S)*-2-(3,4-Dichlorphenyl)-4-{4-[(2*R*,3*S*,4*S*)-2,3,4,5-tetrahydroxy-1-(3-thienyl)pentyl]-1-piperazinyl}-butyl)-N-methylbenzamid;
N-(*(2S)*-2-(3,4-Dichlorphenyl)-4-{4[2*S*,3*R*,4*R*)-2,3,4,5-tetrahydroxyacetyl-1-(3-thienyl)pentyl]-1-piperazinyl}-butyl)-N-methylbenzamid,
N-(*(2S)*-2-(3,4-Dichlorphenyl)-4-{4[(2*S*,3*R*)-2,3,4-trihydroxy-1-(3-thienyl)butyl]-1-piperazinyl}-butyl)-N-methylbenzamid und
N-(*(2S)*-2-(3,4-Dichlorphenyl)-4-{2-furyl[(4*S*)-2-thioxo-1,3-dioxolan-4-yl]methyl}-1-piperazinyl)-butyl]-N-methylbenzamid.

7. Arzneimittel, enthaltend eine pharmakologisch wirksame Menge einer Verbindung gemäß Anspruch 1 und übliche pharmazeutische Hilfs- und/oder Trägerstoffe.

8. Verwendung von Verbindungen gemäß Anspruch 1 zur Herstellung pharmazeutischer Zubereitungen für die Behandlung und/oder Prophylaxe von mit erhöhter Schmerzempfindlichkeit und/oder gestörter Stuhlpassage im Kolonbereich verbundenen funktionellen oder entzündlichen Störungen der unteren Darmwege in Säugetieren und Menschen.

9. Verwendung nach Anspruch 8, worin die Störung IBS ist.

10. Verfahren zur Herstellung von Verbindungen der allgemeinen Formel I, 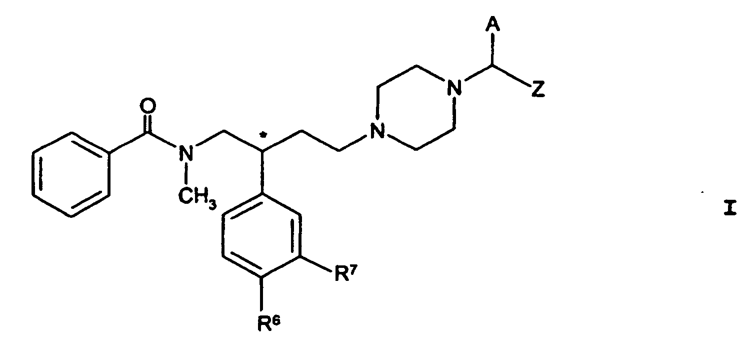 worin
A Naphthyl, gegebenenfalls durch Hydroxy substituiertes Phenyl, mono- oder bicyclisches Heteroaryl oder gegebenenfalls durch Phenyl substituiertes C₃₋₆-Alkenyl bedeutet,
Z für eine Untergruppe der allgemeinen Formel 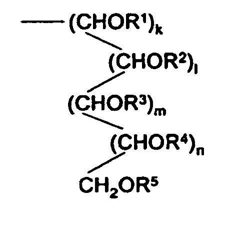 steht, worin
R¹ Wasserstoff oder C₂₋₄-Alkanoyl bedeutet, oder gemeinsam mit einem anderen Substituenten, ausgewählt aus der Gruppe bestehend aus R², R³, R⁴ und R⁵, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
R² Wasserstoff oder C₂₋₄-Alkanoyl bedeutet, oder gemeinsam mit einem anderen Substituenten, ausgewählt aus der Gruppe bestehend aus R¹, R³, R⁴ und R⁵, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
R³ Wasserstoff oder C₂₋₄-Alkanoyl bedeutet, oder gemeinsam mit einem anderen Substituenten, ausgewählt aus der Gruppe bestehend aus R¹, R², R⁴ und R⁵, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
R⁴ Wasserstoff oder C₂₋₄-Alkanoyl bedeutet, oder gemeinsam mit einem anderen Substituenten, ausgewählt aus der Gruppe bestehend aus R¹, R², R³ und R⁵, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
R⁵ Wasserstoff oder C₂₋₄-Alkanoyl bedeutet, oder gemeinsam mit einem anderen Substituenten ausgewählt aus der Gruppe bestehend aus R¹, R², R³ und R⁴, einen über Carbonyl, Thiocarbonyl oder über gegebenenfalls durch C₁₋₄-Alkyl oder C₄₋₅-Alkylen substituiertes Methylen verbrückten 5- oder 6-Ring bilden kann,
k 0 oder 1 bedeutet,
l 0 oder 1 bedeutet,
m 0 oder 1 bedeutet und
n 0 oder 1 bedeutet,
R⁶ Halogen oder Wasserstoff bedeutet,
R⁷ Halogen oder Wasserstoff bedeutet,
sowie deren physiologisch verträglichen Säureadditionssalzen, **dadurch gekennzeichnet, daß** man eine Verbindung der allgemeinen Formel II, 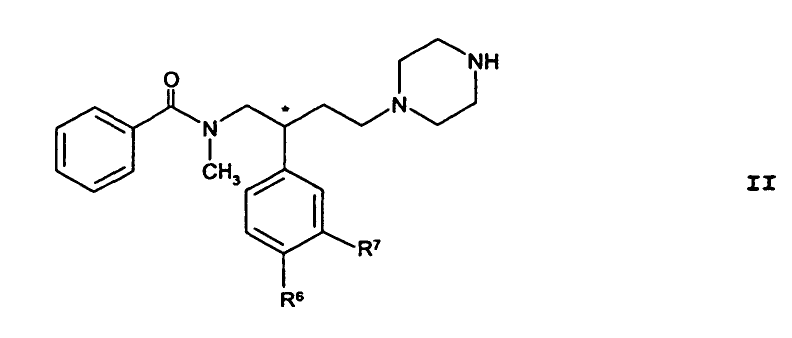 worin R⁶ und R⁷ obige Bedeutungen besitzen, mit einer Verbindung der allgemeinen Formel III,
A-B(OH)₂ **III**
worin A obige Bedeutung besitzt, und mit einer Verbindung der allgemeinen Formel IV, 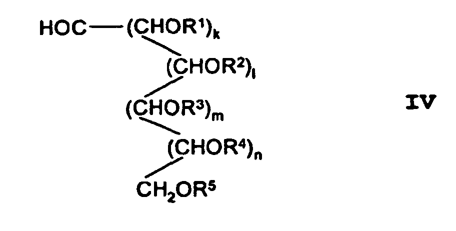 worin R¹, R², R³, R⁴, R⁵, k, l, m und n obige Bedeutungen besitzen, umsetzt und eine erhaltene Verbindung der Formel I, worin mindestens ein Substituent, ausgewählt aus R¹, R², R³, R⁴ und R⁵, Wasserstoff bedeutet, anschließend gewünschtenfalls durch Umsetzung mit einer Verbindung der allgemeinen Formel VIII,
R⁸-COOH (VIII)
worin R⁸ die Bedeutung geradkettiges oder verzweigtes Alkyl mit 1 bis 3 Kohlenstoffatomen besitzt, in der Untergruppe Z acyliert oder eine erhaltene Verbindung der Formel I, worin mindestens zwei Substituenten, ausgewählt aus R¹, R², R³, R⁴ und R⁵ Wasserstoff bedeuten, anschließend gewünschtenfalls durch Umsetzung mit einem reaktiven Carbonyl- oder Thiocarbonyl-Syntheseäquivalent in der Untergruppe Z carbonyliert beziehungsweise thiocarbonyliert, oder eine erhaltene Verbindung der Formel I, worin mindestens zwei Substituenten, ausgewählt aus R¹, R², R³, R⁴ und R⁵, Wasserstoff bedeuten, durch Umsetzung mit einem Diniederalkylketon oder einem C₅₋₆-Cycloalkylketon in der Untergruppe Z zu einem über gegebenenfalls durch C₁₋₄-Alkyl oder C₄₋₅-Alkylen substituiertes Methylen überbrückten 5-oder 6-Ring-Derivat umsetzt, und eine erhaltene Verbindung der Formel I gewünschtenfalls in ihr Säureadditionssalz überführt oder ein Säureadditionssalz in eine freie Verbindung der Formel I überführt.

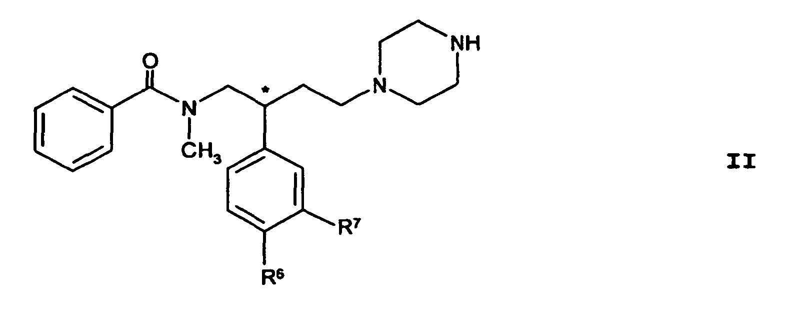

## Claims

1. Compounds of the general formula I, 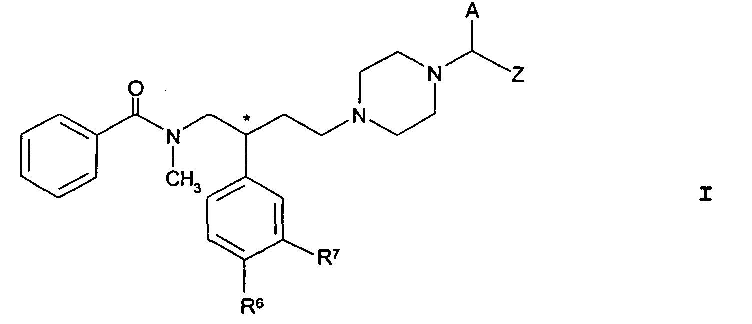 wherein
A is naphthyl, phenyl optionally substituted by hydroxy, mono- or bicyclic heteroaryl or C₃₋₆-alkenyl optionally substituted by phenyl,
Z stands for a subgroup of the general formula 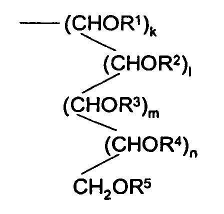 wherein
R¹ is hydrogen or C₂₋₄-alkanoyl, or together with another substituent, selected from the group consisting of R², R³, R⁴ and R⁵, may form a 5- or 6-ring bridged by carbonyl, thiocarbonyl or by methylene optionally substituted by C₁₋₄-alkyl or C₄₋₅-alkylene,
R² is hydrogen or C₂₋₄-alkanoyl, or together with another substituent, selected from the group consisting of R¹, R³, R⁴ and R⁵, may form a 5- or 6-ring bridged by carbonyl, thiocarbonyl or by methylene optionally substituted by C₁₋₄-alkyl or C₄₋₅-alkylene,
R³ is hydrogen or C₂₋₄-alkanoyl, or together with another substituent, selected from the group consisting of R¹, R², R⁴ and R⁵, may form a 5- or 6-ring bridged by carbonyl, thiocarbonyl or by methylene optionally substituted by C₁₋₄-alkyl or C₄₋₅-alkylene,
R⁴ is hydrogen or C₂₋₄-alkanoyl, or together with another substituent, selected from the group consisting of R¹, R², R³ and R⁵, may form a 5- or 6-ring bridged by carbonyl, thiocarbonyl or by methylene optionally substituted by C₁₋₄-alkyl or C₄₋₅-alkylene,
R⁵ is hydrogen or C₂₋₄-alkanoyl, or together with another substituent, selected from the group consisting of R¹, R², R³ and R⁴, may form a 5- or 6-ring bridged by carbonyl, thiocarbonyl or by methylene optionally substituted by C₁₋₄-alkyl or C₄₋₅-alkylene,
k is 0 or 1,
l is 0 or 1,
m is 0 or 1 and
n is 0 or 1,
R⁶ is halogen or hydrogen, and
R⁷ is halogen or hydrogen,
and physiologically compatible acid addition salts of compounds of Formula I.

2. Compounds according to Claim 1, wherein A is thiophene or furan.

3. Compounds according to Claim 1, wherein k is 1 and n is 0.

4. Compounds according to Claim 1, wherein R⁶ and R⁷ each stand for chlorine.

5. Compounds according to Claim 1, wherein the chiral centre *C is in the S configuration.

6. Compounds of Formula I according to one of the preceding claims, which are selected from the group consisting of
*N*-(*(2S)*-2-(3,4-dichlorophenyl)-4-{4-[(2*S*,3*R*,4*R*)-2,3,4,5-tetrahydroxy-1-(3-thienyl)pentyl]-1-piperazinyl}butyl)-*N*-methylbenzamide;
N-(*(2S)*-2-(3,4-dichlorophenyl)-4-{4[(2*S*)-2,3-dihydroxy-1-(2-furyl)propyl]-1-piperazinyl}-butyl)-N-methylbenzamide;
*(2S)*-2-(acetyloxy)-3-{4-[*(3S)*-4-[benzoyl(methyl)amino]-3-(3,4-dichlorophenyl)butyl]-1-piperazinyl}-3-(2-furyl)propylacetate;
N-[*(2S)*-2-(3,4)-dichlorophenyl)-4-(4-{2-furyl[*(4S)*-2-oxo-1,3-dioxolan-4-yl]methyl}-1-piperazinyl)butyl]-N-methylbenzamide;
N-(*(2S)*2-(3,4-dichlorophenyl)-4-{4[(1*S*,2*R*)-2,3-dihydroxy-1-(3-thienyl)propyl]-1-piperazinyl}-butyl)-N-methylbenzamide;
N-(*(2S)*2-(3,4-dichlorophenyl)-4-{4[(2*S*)-2,3-dihydroxy-1-(3-furyl)propyl]-1-piperazinyl}-butyl)-N-methylbenzamide;
N-(*(2S)*-2-(3,4-dichlorophenyl)-4-{4[(2*R*,3*R*,4*R*)-2,3,4,5-tetrahydroxy-1-(3-thienyl)pentyl]-1-piperazinyl}butyl)-N-methylbenzamide;
N-(*(2S)*-2-(3,4-dichlorophenyl)-4-{4[(2*S*,3*R*,4*R*)-2,3,4,5-tetrahydroxy-1-(2-furyl)pentyl]-1-piperazinyl}-butyl)-N-methylbenzamide;
N-(*(2S)*-2-(3,4-dichlorophenyl)-4-{4[(2*R*)-2,3-dihydroxy-1-(3-thienyl)propyl]-1-piperazinyl}-butyl)-N-methylbenzamide;
N-(*(2S)*-2-(3,4-dichlorophenyl)-4-{4[(2*R*,3*S*,4*S*)-2,3,4,5-tetrahydroxy-1-(3-thienyl)pentyl]-1-piperazinyl}butyl)-N-methylbenzamide;
N-(*(2S)*-2-(3,4-dichlorophenyl)-4-{4[2*S*,3*R*,4*R*)-2,3,4,5-tetrahydroxyacetyl-1-(3-thienyl)pentyl]-1-piperazinyl}-butyl)-N-methylbenzamide,
N-(*(2S)*-2-(3,4-dichlorophenyl)-4-{4[(2*S*,3*R*)-2,3,4-trihydroxy-1-(3-thienyl)butyl]-1-piperazinyl}-butyl)-N-methylbenzamide and
N-(*(2S)*-2-(3,4-dichlorophenyl)-4-{2-furyl[(4*S*)-2-thioxo-1,3-dioxolan-4-yl]methyl}-1-piperazinyl)-butyl]-N-methylbenzamide.

7. Medicament, containing a pharmacologically effective quantity of a compound according to Claim 1 and conventional pharmaceutical auxiliaries and/or excipients.

8. The use of compounds according to Claim 1 for the preparation of pharmaceutical preparations for the treatment and/or prophylaxis of functional or inflammatory disorders in the lower intestinal tracts in mammals and humans which involve increased sensitivity to pain and/or impaired stool passage in the colon region.

9. The use according to Claim 8, wherein the disorder is IBS.

10. A process for the preparation of compounds of the general formula I, 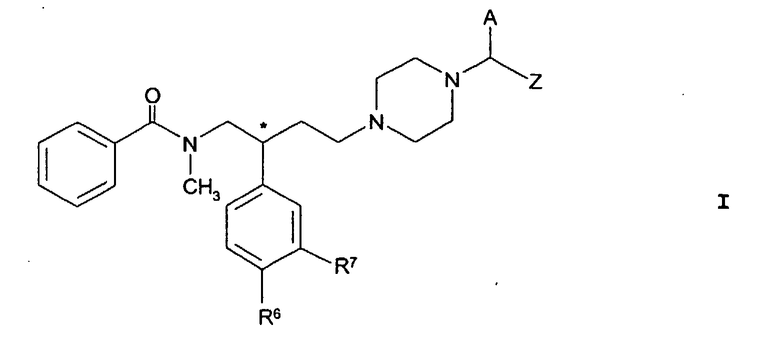 wherein
A is naphthyl, phenyl optionally substituted by hydroxy, mono- or bicyclic heteroaryl or C₃₋₆-alkenyl optionally substituted by phenyl,
Z stands for a subgroup of the general formula 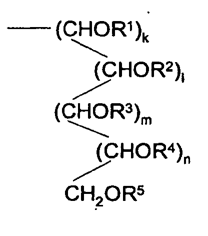 wherein
R¹ is hydrogen or C₂₋₄-alkanoyl, or together with another substituent, selected from the group consisting of R², R³, R⁴ and R⁵, may form a 5- or 6-ring bridged by carbonyl, thiocarbonyl or by methylene optionally substituted by C₁₋₄-alkyl or C₄₋₅-alkylene,
R² is hydrogen or C₂₋₄-alkanoyl, or together with another substituent, selected from the group consisting of R¹, R³, R⁴ and R⁵, may form a 5- or 6-ring bridged by carbonyl, thiocarbonyl or by methylene optionally substituted by C₁₋₄-alkyl or C₄₋₅-alkylene,
R³ is hydrogen or C₂₋₄-alkanoyl, or together with another substituent, selected from the group consisting of R¹, R², R⁴ and R⁵, may form a 5- or 6-ring bridged by carbonyl, thiocarbonyl or by methylene optionally substituted by C₁₋₄-alkyl or C₄₋₅-alkylene,
R⁴ is hydrogen or C₂₋₄-alkanoyl, or together with another substituent, selected from the group consisting of R¹, R², R³ and R⁵, may form a 5- or 6-ring bridged by carbonyl, thiocarbonyl or by methylene optionally substituted by C₁₋₄-alkyl or C₄₋₅-alkylene,
R⁵ is hydrogen or C₂₋₄-alkanoyl, or together with another substituent, selected from the group consisting of R¹, R², R³ and R⁴, may form a 5- or 6-ring bridged by carbonyl, thiocarbonyl or by methylene optionally substituted by C₁₋₄-alkyl or C₄₋₅-alkylene,
k is 0 or 1,
l is 0 or 1,
m is 0 or 1 and
n is 0 or 1,
R⁶ is halogen or hydrogen,
R⁷ is halogen or hydrogen,
and the physiologically compatible acid.addition salts thereof, **characterised in that** a compound of the general formula II, 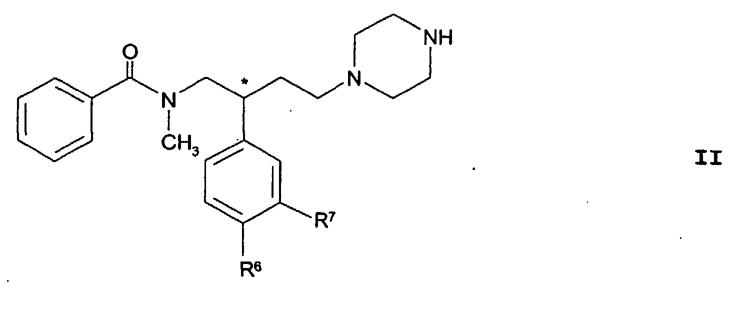 wherein R⁶ and R⁷ have the above meanings, is reacted with a compound of the general formula III,
A-B(OH)₂ **III**
wherein A has the above meaning, and with a compound of the general formula IV, 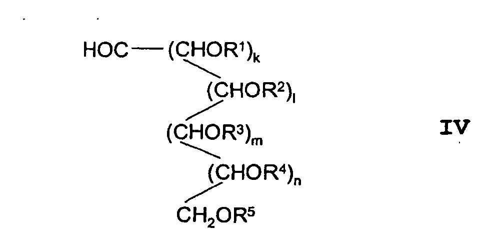 wherein R¹, R², R³, R⁴, R⁵, k, l, m und n have the above meanings, and a resulting compound of Formula I, wherein at least one substituent, selected from R¹, R², R³, R⁴ and R⁵, is hydrogen, then if desired is acylated in the subgroup Z by reacting with a compound of the general formula VIII,
R⁸-COOH (VIII)
wherein R⁸ has the meaning of straight-chain or branched alkyl with 1 to 3 carbon atoms, or a resulting compound of Formula I, wherein at least two substituents, selected from R¹, R², R³, R⁴ and R⁵ are hydrogen, then if desired is carbonylated or thiocarbonylated respectively in the subgroup Z by reacting with a reactive carbonyl- or thiocarbonyl synthesis equivalent, or a resulting compound of Formula I wherein at least two substituents; selected from R¹, R², R³, R⁴ and R⁵, are hydrogen, by reacting with a di-lower alkylketone or a C₅₋₆-cycloalkylketone in the subgroup Z to form a 5-or 6-ring derivative bridged by methylene optionally substituted by C₁₋₄-alkyl or C₄₋₅-alkylene, and a resulting compound of Formula I if desired is converted into its acid addition salt or an acid addition salt is converted into a free compound of Formula I.

## Revendications

1. Composés de formule générale I 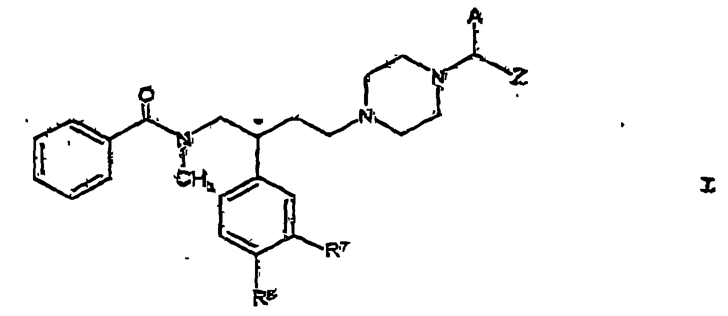 dans laquelle
A est un groupe naphtyle, phényle éventuellement à substitution hydroxy, hététoaryle monocyclique ou bicyclique, ou alcényle en C₃₋₆ éventuellement à substitution phényle,
Z est un sous-groupe de formule générale 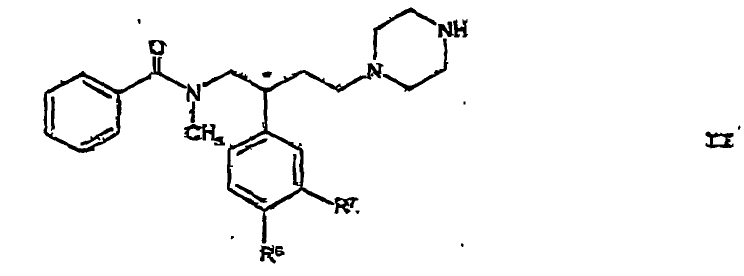 dans laquelle
R¹ est un atome d'hydrogène ou un groupe alcanoyle en C₂₋₄, ou, pris avec un autre substituant choisi dans l'ensemble consistant en R², R³, R⁴ et R⁵, peut former un radical cyclique à 5 ou 6 chaînons, ponté par un radical carbonyle, thiocarbonyle ou par un radical méthylène, éventuellement à substitution alkyle en C₁₋₄ ou alkylène en C₄₋₅,
R² est un atome d'hydrogène ou un groupe alcanoyle en C₂₋₄, ou, pris avec un autre substituant choisi dans l'ensemble consistant en R¹, R³, R⁴ et R⁵, peut former un radical cyclique à 5 ou 6 chaînons, ponté par un radical carbonyle, thiocarbonyle ou par un radical méthylène, éventuellement à substitution alkyle en C₁₋₄ ou alkylène en C₄₋₅,
R³ est un atome d'hydrogène ou un groupe alcanoyle en C₂₋₄, ou, pris avec un autre substituant choisi dans l'ensemble consistant en R¹, R², R⁴ et R⁵, peut former un radical cyclique à 5 ou 6 chaînons, ponté par un radical carbonyle, thiocarbonyle ou par un radical méthylène, éventuellement à substitution alkyle en C₁₋₄ ou alkylène en C₄₋₅,
R⁴ est un atome d'hydrogène ou un groupe alcanoyle en C₂₋₄, ou, pris avec un antre substituant choisi dans l'ensemble consistant en R¹, R², R³ et R⁵, peut former un radical cyclique à 5 ou 6 chaînons, ponté par un radical carbonyle, thiocarbonyle ou par un radical méthylène, éventuellement à substitution alkyle en C₁₋₄ ou alkylène en C₄₋₅,
R⁵ est un atome d'hydrogène ou un groupe alcanoyle en C₂₋₄, ou, pris avec un autre substituant choisi dans l'ensemble consistant en R¹, R², R³ et R⁴, peut former un radical cyclique à 5 ou 6 chaînons, ponté par un radical carbonyle, thiocarbonyle ou par un radical méthylène, éventuellement à substitution alkyle en C₁₋₄ ou alkylène en C₄₋₅,
k vaut 0 ou 1,
l vaut 0 ou 1,
m vaut 0 ou 1, et
n vaut 0 ou 1
R⁶ est un halogène ou l'hydrogène, et
R⁷ est un halogène ou l'hydrogène,
ainsi que les sels d'addition avec un acide, tolérés d'un point de vue physiologique, des composés de formule I.

2. Composés selon la revendication 1, dans lesquels A est le thiophène ou le furanne.

3. Composés selon la revendication 1, dans lesquels k vaut 1 et n vaut 0.

4. Composés selon la revendication 1, dans lesquels R⁶ et R⁷ représentent chacun le chlore.

5. Composés selon la revendication 1, dans lesquels le centre de chiralité *C présente la configuration S.

6. Composés de formule I selon l'une des revendications précédentes, qui sont choisis dans l'ensemble consistant en les composés suivants :
N-((2S)-2-(3,4-dichlorophényl)-4-{4-[(2S,3R,4R)-2,3,4,5-tétrahydroxy-1-(3-thiényl)pentyl]-1-pipérazinyl}butyl)-N-méthylbenzamide ;
N-((2S)-2-(3,4-dichlorophényl)-4-{4[(2S)-2,3-dihydroxy-1-(2-furyl)propyl]-1-pipérazinyl}-butyl)-N-méthylbenzamide ;
Acétate de (2S)-2-(acétyloxy)-3-{4-[(3S)-4-[benzoyl(méthyl)-amino]-3-(3,4-dichlorophényl)butyl]-1-pipérazinyl}-3-(2-furyl)propyle ;
N-[(2S)-2-(3,4-dichlorophényl)-4-(4-{2-furyl[(4S)-2-oxo-1,3-dioxolanne-4-yl]méthyl}-1-pipérazinyl)butyl]-N-méthyl-benzamide ;
N-((2S)-2-(3,4-dichlorophényl)-4-{4[(1S,2R)-2,3-dihydroxy-1-(3-thiényl)propyl]-1-pipérazinyl}-butyl)-N-méthylbenzamide ;
N-((2S)-2-(3,4-dichlorophényl)-4-{4[(2S)-2,3-dihydroxy-1-(3-furyl)propyl]-1-pipérazinyl}-butyl)-N-méthylbenzamide ;
N-((2S)-2-(3,4-dichlorophényl)-4-{4[(2R,3R,4R)-2,3,4,5-tétrahydroxy-1-(3-thiényl)pentyl]-1-pipérazinyl}butyl)-N-méthylbenzamide ;
N-((2S)-2-(3,4-dichlorophényl)-4-{4[(2S,3R,4R)-2,3,4,5-tétrahydroxy-1-(2-furyl)pentyl]-1-pipérazinyl}butyl)-N-méthylbenzamide ;
N-((2S)-2-(3,4-dichlorophényl)-4-{4[(2R)-2,3-dihydroxy-1-(3-thiényl)propyl]-1-pipérazinyl}-butyl)-N-méthylbenzamide ;
N-((2S)-2-(3,4-dichlorophényl)-4-{4-[(2R,3S,4S)-2,3,4,5-tétrahydroxy-1-(3-thiényl)pentyl]-1-pipérazinyl}-butyl)-N-méthylbenzamide ;
N-((2S)-2-(3,4-dichlorophényl)-4-{4[(2S,3R,4R)-2,3,4,5-tétrahydroxyacétyl-1-(3-thiényl)pentyl]-1-pipérazinyl}-butyl)-N-méthylbenzamide ;
N-((2S)-2-(3,4-dichlorophényl)-4-{4[(2S,3R)-2,3,4-trihydroxy-1-(3-thiényl)butyl]-1-pipérazinyl}-butyl)-N-méthylbenzamide, et
N-((2S)-2-(3,4-dichlorophényl)-4-{2-furyl[(4S)-2-(thioxo-1,3-dioxolanne-4-yl]méthyl}-1-pipérazinyl)-butyl]-N-méthylbenzamide.

7. Médicament contenant une quantité pharmacologiquement efficace d'un composé selon la revendication 1 et des adjuvants et/ou excipients pharmaceutiques usuels.

8. Utilisation de composés selon la revendication 1 pour fabriquer des préparations pharmaceutiques destinées au traitement et/ou à la prophylaxie de troubles fonctionnels ou inflammatoires, liés à une augmentation de la sensibilité à la douleur et/ou à une perturbation du passage des selles dans la zone du côlon, des voies intestinales inférieures chez les mammifères et les humains.

9. Utilisation selon la revendication 8, où le trouble est le syndrome IBS.

10. Procédé de préparation de composés de formule générale I 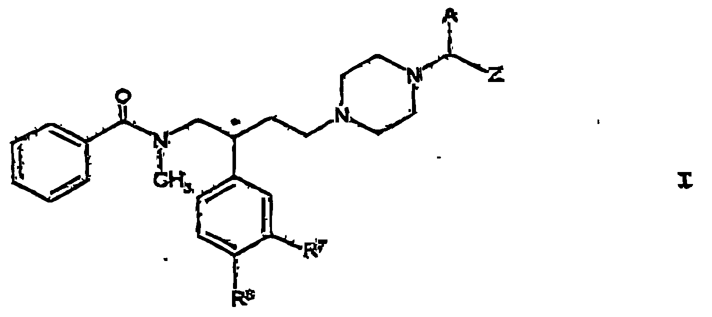 dans laquelle
A est un groupe naphtyle, phényle éventuellement à substitution hydroxy, hétéroaryle monocyclique ou bicyclique, ou alcényle en C₃₋₆ éventuellement à substitution phényle,
Z est un sous-groupe de formule générale 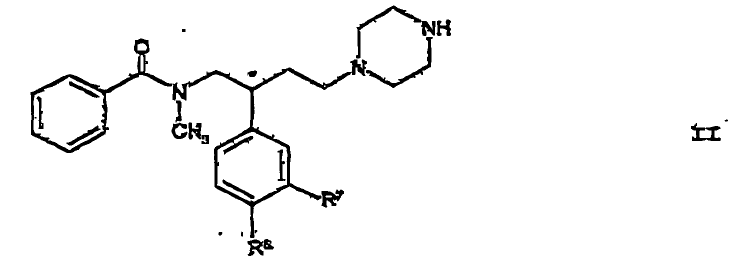 dans laquelle
R¹ est un atome d'hydrogène ou un groupe alcanoyle en C₂₋₄, ou, pris avec un autre substituant choisi dans l'ensemble consistant en R², R³, R⁴ et R⁵, peut former un radical cyclique à 5 ou 6 chaînons, ponté par un radical carbonyle, thiocarbonyle ou par un radical méthylène, éventuellement à substitution alkyle en C₁₋₄ ou alkylène en C₄₋₅,
R² est un atome d'hydrogène ou un groupe alcanoyle en C₂₋₄, ou, pris avec un autre substituant choisi dans l'ensemble consistant en R¹, R³, R⁴ et R⁵, peut former un radical cyclique à 5 ou 6 chaînons, ponté par un radical carbonyle, thiocarbonyle ou par un radical méthylène, éventuellement à substitution alkyle en C₁₋₄ ou alkylène en C₄₋₅,
R³ est un atome d'hydrogène ou un groupe alcanoyle en C₂₋₄, ou, pris avec un autre substituant choisi dans l'ensemble consistant en R¹, R², R⁴ et R⁵, peut former un radical cyclique à 5 ou 6 chaînons, ponté par un radical carbonyle, thiocarbonyle ou par un radical méthylène, éventuellement à substitution alkyle en C₁₋₄ ou alkylène en C₄₋₅,
R⁴ est un atome d'hydrogène ou un groupe alcanoyle en C₂₋₄, ou, pris avec un autre substituant choisi dans l'ensemble consistant en R¹, R², R³ et R⁵, peut former un radical cyclique à 5 on 6 chaînons, ponté par un radical carbonyle, thiocarbonyle ou par un radical méthylène, éventuellement à substitution alkyle en C₁₋₄ ou alkylène en C₄₋₅,
R⁵ est un atome d'hydrogène ou un groupe alcanoyle en C₂₋₄, ou, pris avec un autre substituant choisi dans l'ensemble consistant en R¹, R², R³ et R⁴, peut former un radical cyclique à 5 ou 6 chaînons, ponté par un radical carbonyle, thiocarbonyle ou par un radical méthylène, éventuellement à substitution alkyle en C₁₋₄ ou alkylène en C₄₋₅,
k vaut 0 ou 1,
l vaut 0 ou 1,
m vaut 0 ou 1, et
n vaut 0 ou 1
R⁶ est un halogène ou l'hydrogène, et
R⁷ est un halogène ou l'hydrogène,
ainsi que les sels d'addition avec un acide, tolérés d'un point de vue physiologique, **caractérisé en ce qu'**on fait réagir un composé de formule générale II 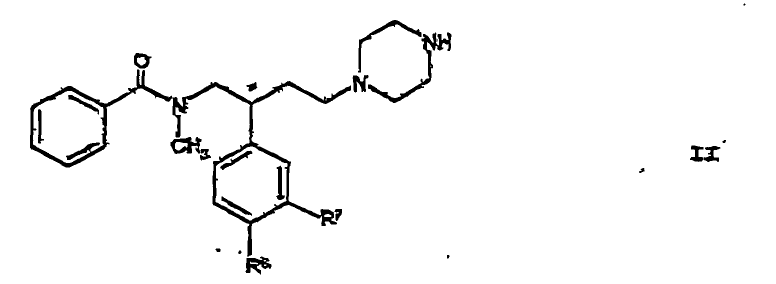 où R⁶ et R⁷ ont les significations ci-dessus, avec un composé de formule générale III,
A-B(OH)₂ III
où A a la signification ci-dessus, et avec un composé de formule générale IV 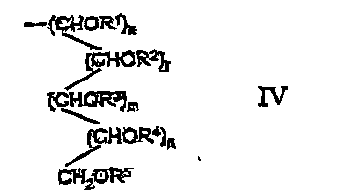 dans laquelle R¹, R², R³, R⁴, R⁵, k, l, m et n ont les significations ci-dessus, puis on acyle éventuellement dans le sous-groupe Z un composé ainsi obtenu de formule I dans laquelle au moins un substituant choisi parmi R¹, R², R³, R⁴ et R⁵ est l'hydrogène, par réaction avec un composé de formule générale VIII
R⁸-COOH (VIII)
où R⁸ représente un radical alkyle à chaîne droite ou ramifiée ayant de 1 à 3 atomes de carbone ; ou encore on soumet éventuellement ensuite à une carbonylation ou à une thiocarbonylation dans le sous-groupe Z un composé ainsi obtenu de formule I dans laquelle au moins deux substituants choisis parmi R¹, R², R³, R⁴ et R⁵ sont l'hydrogène, par réaction avec un équivalent de synthèse carbonylé ou thiocarbonylé réactif ; ou encore on fait réagir dans le sous-groupe Z un composé ainsi obtenu de formule I dans laquelle au moins deux substituants choisis parmi R¹, R², R³, R⁴ et R⁵ sont l'hydrogène, par réaction avec une di(alkyle inférieur)cétone ou une (cycloalkyle en C₅₋₆)cétone pour former un dérivé à 5 ou 6 chaînons, ponté par un radical méthylène, éventuellement à substitution alkyle en C₁₋₄ ou alkylène en C₄₋₅, et, si on le souhaite, on convertit un composé ainsi obtenu de formule I en son sol d'addition avec un acide, ou encore on convertit un sel d'addition avec un acide en un composé libre de formule I.
